(19)

**Europäisches Patentamt**
**European Patent Office**
**Office européen des brevets**

(11) **EP 4 595 948 A1**

(12) **EUROPEAN PATENT APPLICATION**

(43) Date of publication:
**06.08.2025 Bulletin 2025/32**

(21) Application number: **24382099.0**

(22) Date of filing: **31.01.2024**

(51) International Patent Classification (IPC):
*A61K 9/00* (2006.01)    *A61K 9/51* (2006.01)
*A61K 9/10* (2006.01)

(52) Cooperative Patent Classification (CPC):
**A61K 9/5115; A61K 9/0019; A61K 9/5169;**
A61K 9/10

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
  • **Universidad de Granada**
    **18071 Granada (ES)**
  • **Universidad De Zaragoza**
    **50009 Zaragoza (ES)**
  • **Università Degli Studi Di Verona**
    **37129 Verona (IT)**

(72) Inventors:
  • **JIMÉNEZ LÓPEZ, Concepción**
    **18071 Granada (ES)**
  • **MAQUEDA ABREU, Mercedes**
    **18071 Granada (ES)**
  • **MONTALBÁN LÓPEZ, Manuel**
    **18071 Granada (ES)**
  • **IGLESIAS SALTO, Guillermo Ramón**
    **18071 Granada (ES)**

  • **FERNÁNDEZ RODRÍGUEZ, Matilde**
    **18071 Granada (ES)**
  • **CARRASCO JIMÉNEZ, María Paz**
    **18071 Granada (ES)**
  • **PERDUCA, Massimiliano**
    **37134 Verona (IT)**
  • **AÍNSA CLAVER, José Antonio**
    **50009 Zaragoza (ES)**
  • **JABALERA RUZ, Ylenia**
    **18071 Granada (ES)**
  • **GAGLIO, Salvatore Calogero**
    **37134 Verona (IT)**
  • **JIMÉNEZ CARRETERO, Mónica**
    **18071 Granada (ES)**
  • **LÁZARO CALLEJÓN, Marina**
    **18071 Granada (ES)**
  • **POZO GUALDA, Tamara**
    **18071 Granada (ES)**
  • **SOLA LEYVA, Alberto**
    **18071 Granada (ES)**

(74) Representative: **Hoffmann Eitle**
    **Hoffmann Eitle S.L.U.**
    **Paseo de la Castellana 140, 3a planta**
    **Edificio LIMA**
    **28046 Madrid (ES)**

(54) **BMNPS AND NANOASSEMBLIES THEREOF**

(57)    The present invention refers to a composition comprising BMNPs (Biomimetic magnetic nanoparticles) or BMNP nanoassemblies, characterized in that said BMNPs are disaggregated or dispersed. Alternatively, the first aspect of the present invention refers to a composition comprising: (i) a substantially pure mineral phase of superparamagnetic magnetite, (ii) the MamC protein, and (iii) optionally, the Mms6 and/or Mms7 protein; wherein, at least components (i) and (ii) form superparamagnetic magnetic nanoparticles containing up to 20 wt% of MamC, with a mean particle size between 30 and 120 nm, preferably between 30 and 50 nm, more preferably about $39 \pm 7$ nm, characterized in that said superparamagnetic magnetic nanoparticles containing MamC are disaggregated or dispersed.

EP 4 595 948 A1

**Description**

**TECHNICAL FIELD OF THE INVENTION**

The present invention relates to the medical field, more particularly to compositions comprising biomimetic magnetic nanoparticles and uses thereof.

**BACKGROUND ART**

Antimicrobial therapy is gaining a great interest, prompted by the increasing generation of antibiotic resistances that are not paired with the discovery of new therapeutic strategies. Directed chemotherapy has emerged as a promising alternative to systemic treatments, allowing the selective delivery of the therapeutic agent to the target. In some cases, directed chemotherapy also allows the localized *in situ* combination of several therapeutic treatments, which synergy may amplify the efficiency of the individual ones, therefore maximizing the bacterial eradication while minimizing resistance appearance and concomitantly reduce undesired side systemic effects. This strategy has been largely explored in the case of cancer, and some formulations have been proven effective against localized tumours, also minimizing secondary effects. In the case of bacterial infections, this strategy is much less explored. Wu et al. (2019), using Zeolitic imidazolate frameworks-8, showed the potential of combining a photothermic-chemical treatment for murine subcutaneous abscess induced by methicillin-resistant *Staphylococcus aureus.* Wang et al. (2020) developed a graphene based dual-targeted platform for synergistic chemo-photothermal therapy against multidrug-resistant Gram-negative bacteria and their biofilms. If successful, directed chemotherapy combined with other therapies would allow to reduce the required antibacterial dose, so lowering of the chances of developing and/or generating bacterial resistances. However, these formulations have the drawback of the efficient guiding to the target.

Within the many nanocarriers developed, magnetic nanoparticles (MNP) have become attractive candidates. Their large surface area allows to carry relatively large amounts of the relevant molecule and their magnetic properties allow an external guidance and/or concentration at the target, plus the combination of therapies such as chemotherapy and magnetic hyperthermia. However, the already commercialized inorganic MNPs usually fail (El-Boubbou, 2018), so choosing an optimized magnetic nanocarrier is the "bottle neck" for this use. In fact, commercialized MNPs present two main drawbacks that difficult their use, one linked to their relatively small size (around 10-20 nm), which results in a small magnetic moment per particle, and the other related to their surface charge, that is fairly low (or neutral) at physiological pH (Garcia-Rubia et al., 2018). Therefore, using superparamagnetic magnetic nanocarriers with larger sizes (20-120 nm) would represent a great advantage for most applications. In the context of surface properties, inorganic magnetic nanoparticles usually have an isoelectric point (iep) close to 7, so they need to be coated with different polymers to allow functionalization which shelters the already non-optimal magnetic properties, then jeopardizing their use even further (El-Boubbou, 2018). There are different brands that sell this type of superparamagnetic magnetite and maghemite nanoparticles particles: Ocean Nanotech, Sigma, Das Nano, Micromod, nanoComposix europe, NVIGEN (MagVigen), Cytodiagnostic, among others.

These issues are overcome by biomimetic magnetosome-like magnetic nanoparticles (BMNPs), inspired by magnetotactic bacteria. These BMNPs are chemically produced by introducing a magnetosome protein from *Magnetococcus marinus* MC-1, MamC (expressed as a recombinant protein), in the reaction mixture from which magnetite nucleates and grows, at room temperature and atmospheric pressure (Valverde-Tercedor et al., 2015). MamC chelates Fe cations and acts as template for magnetite nucleation (Ubago et al., 2019), producing magnetic nanoparticles different in size, surface charge and magnetic properties from those chemically produced (MNPs) (Garcia-Rubia et al., 2018). MamC-mediated biomimetic magnetic nanoparticles have proven to be suitable nanocarriers for a directed combined therapy. In terms of size, BMNPs are larger (~35-40 nm) than MNPs (<20nm), which allow BMNPs to be superparamagnetic and to display a larger magnetic moment per particle than that displayed by MNPs. This characteristic ensures an enhanced magnetic response once an external magnetic field is applied for guidance and/or concentration, and favors a non-magnetic behavior of BMNPs in the absence of an external magnetic field which prevents BMNPs aggregation.

In terms of surface charge, the advantage of using BMNPs versus MNPs is mainly related to the greater ease in the process of functionalization. MNPs usually have an isoelectric point close to 7, thus they need to be coated to successfully bind the relevant molecule and to keep stable the nanoassembly at physiological pH. Instead, in the case of BMNPs, MamC confers to the nanoparticles surface functional groups, which switch their isoelectric point to ~4.4. This is important, since being the nanoparticle negatively charged at physiological pH, drug binding by electrostatic interaction and nanoassembly stability at this pH value is ensured, while drug release is triggered in acidic environments as BMNPs become uncharged, BMNPs behaving as a stimulus-response drug delivery system. Furthermore, BMNPs are hyperthermia agents, able to locally raise the temperature to the hyperthermia therapeutic range (41-45°C) following upon the application of an alternating magnetic field (AMF), which, moreover, further increases drug release at the target.

El-Boubbou, K. Magnetic iron oxide nanoparticles as drug carriers: preparation, conjugation and delivery.

Nanomedicine 2018, 13, 929-952. 10.2217/nnm-2017-0320.

Garcia Rubia, G.; Peigneux, A.; Jabalera, Y; Puerma, J.; Oltolina, F.; Elert, K.; Colangelo, D.; Gómez Morales, J.; Prat, M.; Jimenez-Lopez, C. pH-Dependent adsorption release of doxorubicin on MamC-Biomimetic Magnetite Nanoparticles. Langmuir 2018, 34, 13713-13724., 10.1021/acs.langmuir.8b03109.

Ubago-Rodriguez, A.; Casares Atienza, S.; Fernández-Vivas, A.; Peigneux, A.; Jabalera, Y; De La Cuesta-Rivero, M.; Jimenez-Lopez, C.; Azuaga Fortes, A.I. Structure-Function of MamC Loop and Its Effect on the in Vitro Precipitation of Biomimetic Magnetite Nanoparticles. Crystal Growth and Design 2019, 19 (5), 2927-2935. 10.1021/acs.cgd.9b00150.

Valverde-Tercedor, C.; Montalbán-López, M.; Perez-Gonzalez, T.; Sanchez-Quesada, M,S,; Prozorov, T.; Pineda-Molina, E.; Fernandez-Vivas, M.A.; Rodriguez-Navarro, A.B.; Jimenez-Lopez C. Size control of in vitro synthesized magnetite crystals by the MamC protein of Magnetococcus marinus strain MC-1. Appl. Microbiol. Biotechnol. 2015, 99, 5109-5121. 10.1007/s00253-014-6326-y.

Wang, H.; Zhao, B.; Dong, W.; Zhong, Y.; Zhang, X.; Gong, Y; Zhan, R.; Xing, M.; Zhang, J.; Luo, G.; et al. A Dual-Targeted Platform Based on Graphene for Synergistic Chemo-Photothermal Therapy against Multidrug-Resistant Gram-Negative Bacteria and Their Biofilms. Chemical Engineering Journal 2020, 393, 124595, 10.1016/j.cej.2020.124595.

Wu, B.; Fu, J.; Zhou, Y; Shi, Y; Wang, J.; Feng, X.; Zhao, Y; Zhou, G.; Lu, C.; Quan, G.; et al.Metal-Organic Framework-Based Chemo-Photothermal Com-binational System for Precise, Rapid, and Efficient Antibacterial Therapeutics. Pharmaceutics 2019, 11, 463, .10.3390/pharmaceutics11090463.

## BRIEF DESCRIPTION OF THE FIGURES

**Figure 1** shows HepG2 cells: (A) non treated (without BMNPs), (B) incubated with BMNPs from Group I, and (C) incubated with BMNPs from Group II. Human hepatoblastoma cell line (HepG2) under inverted optical microscope (10X) without BMNPs (A), and with BMNPs before (B) and after (C) the disaggregation protocol. All the images have identical resolution.

**Figure 2.** Growth of *Mycobacterium tuberculosis* in treated cultures relative to untreated cultures, at different concentrations of bacteriocin AS-48. In formulations containing AS-48, its concentration has been taken as the reference for representing the plot.

**Figure 3.** Intracellular growth of *M. tuberculosis* H37Rv-Luc strain inside THP-1 macrophages upon treatment with bacteriocin AS-48 free or immobilized.

**Figure 4.** Cytotoxicity. THP-1 macrophage viability after 72h incubation with culture medium (control), BMNPs, free AS-48 orAS-48_BMNPs nanoassemblies.

**Figure 5.** Therapy against intracellular *M. tuberculosis.* Combined treatment of immobilized AS-48 with magnetic hyperthermia (MH) (A) and photothermia (PT) (B). Interval times: 0 (before adding the treatment), 72h (after incubation with the treatment), After MH or PT (immediately after the 20 min application of MH or PT), 24h post-MH or PT (after 24h incubation to recover from treatment).

**Figure 6.** Effect of the nanoformulations on biofilms formed by *Enterococcus faecalis* (A) and *Pseudomonas aeruginosa* (B).

## DESCRIPTION OF EMBODIMENTS

### Definitions

The terms "treatment' and "therapy", as used in this specification, refer to a set of hygienic, pharmacological, surgical, and/or physical protocols used with the intent to cure and/or alleviate a disease and/or symptoms with the goal of improving the state of health. The terms "treatment" and "therapy" include preventive and curative methods, since both are directed to the maintenance or reestablishment of health of an individual or animal. Regardless of the origin of the symptoms, disease and disability, the administration of a suitable medicament to alleviate and/or cure a health problem should be interpreted as a form of treatment or therapy within the context of this specification.

The term "therapeutically effective amount" refers to the amount of compound in a composition or formulation which has a therapeutic effect, and which is able to treat a disease.

As used herein, "pharmaceutically acceptable carrier" or "pharmaceutically acceptable diluent" refers to any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are not toxic for the subject at the dosages and concentrations employed and, without limiting the scope of the present invention, include: water, buffers like HEPES or other buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thiosulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the characteristics of the pharmaceutical composition.

The term "therapeutic agent" refers to any substance or stimuli which can be used to treat and/or prevent a disease when used in therapeutically effective amounts. The therapeutic agent may be a small chemical molecule (for example, a doxorubicin, antihistamine, etc.), biological molecule (for example, a therapeutic protein), nucleic acid (for example, siRNA, gRNA for CRISPR/Cas9, etc.), or physical stimuli mediated by an organic or inorganic composition such as hyperthermia mediated by hyperthermia agents. The term "magnetic hyperthermia" refers to local heating produced by magnetic hyperthermia agents following upon exposure to time-varying magnetic fields. The term "photothermia" refers to local heating produced by photothermal agents following upon exposure to electromagnetic waves of wavelengths in the visible and infrared spectrum.

The term "chemotherapeutic agent" refers to any drug which can be used, either by itself or combined, to treat or prevent cancer, bacterial, viral and/or parasitic infections. Non-limiting examples include: Actinomycin. All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine and Vinorelbine. Antibiotics: aminoglycosides (e.g. gentamicin, amikacin, tobramycin, plazomicin), carbapenems (e.g. imipenem, meropenem), cephalosporins (e.g. cephalexin, cefdinir, ceftazidime, cefepime), fluoroquinolones (e.g. nalidixic acid, ciprofloxacin, levofloxacin), glycopeptides (e.g. vancomycin, teicoplanin), lipopeptides (e.g. daptomycin), polymyxins (e.g. colistin, polymyxin B), macrolides (e.g. erythromycin, clarithromycin, azithromycin), monobactams (e.g. aztreonam, nocardicin A), oxazolidinones (e.g. linezolid, tedizolid), penicillins (e.g. amoxicillin, piperacillin, oxacillin), rifamycins (e.g. rifampicin, rifabutin, rifamixin), sulfonamides (e.g. sulfadiazine, sulfamethoxazole), pyrimidines (e.g. trimethoprim, pyrimethamine), streptogramins (e.g. pristinamycin, dalfopristin, quinupristin), tetracyclines (e.g. doxycyline, tigecycline), chloramphenicol, clindamycin and lincomicins, fosfomycin, pleuromutilins (e.g. lefamulin, valnemulin), metronidazole, mupirocin, nitrofurantoin, ethambutol. Antimicrobial peptides: e.g. defensins, cathelicidins, indolicidins, polyphemusins, insect antimicrobial peptides (apidaecins, cecropins, mellitins, proline-rich antimicrobial peptides, protegrins and arginine-rich antimicrobial peptides), plant antimicrobial peptides (thionins, defensins, amphibians and other animal derived antimicrobial peptides (magainins brevinins, ranateurins, bombinins, esculentins, rugosins, temporins, citropins), ribosomally produced and posttranslationally modified peptides (RiPPs; circular bacteriocins such as AS-48 or its derivatives, cyanobactins, bottromycins, lasso-peptides, sactipeptides among others), bacteriocins, non-ribosomally synthesized peptides (gramicidins, polymyxins, tyrocidines, daptomycin). Peptidomimetics (e.g. omiganan, lytixar, brilacidin, pexiganan), synthetic antimicrobial peptides (novispririn, iseganan, RR, HB1345, RRIKA, DASamP1 , HHC-10, HHC-36), peptoids/ampetoids (ampetoid 1, GN-2, GN4, peptoid 13, L3, L4, L6, C3, C4, C6) and peptaibols (emerimicin, trichokonins, chilenopeptins, septocylindrins, hyporientalin, trichorzianines, pentadecaibins, leucinostatin). Antimicrobial inorganic salts (e.g. copper and silver salts, among other). Antiseptics such as quaternary ammonium salts (benzalkonium chloride, cetyltrimethylammonium chloride), boric acid, chlorhexidine. Exopolysaccharide-degrading enzymes (such as glucanohydrolases and dispersin B, among others). Quorum sensing Inhibitors (QSI).

The term "substantially pure mineral phase" refers to a mineral phase which mostly consists of a single type of mineral ($\geq 90\%$). In this case, a substantially pure mineral phase of magnetite means that the magnetite crystals do not contain siderite. The substantially pure mineral phase of magnetite may contain small amounts of goethite ($\leq 10\%$) if the pH is raised above 9 during the production process.

The term "superparamagnetism" refers to a form of magnetism which appears in small ferromagnetic or ferrimagnetic nanoparticles. Below a certain size, magnetic nanoparticles can no longer support the static walls of different magnetic domains, behaving like a giant "spin" of magnetic moment. A "superparamagnetic" particle can be free (thermally equilibrated) or blocked (not equilibrated).

The term "inorganic magnetite nanoparticles" or MNPs refers to any magnetic nanoparticle, most preferably magnetite, which is obtained or obtainable through chemical synthesis methods in the absence of any biological agent and/or product.

The term "MAP or MAPs" refers to functional length, functional fragments and variants of magnetosome associate proteins, including, but not restricted to, MamC, Mms6 and Mms7.

The term "MamC" refers to a full-length protein which is derived from the *mamC* gene (NCBI Database, gene accession number ABK44766.1, protein accession Mmc1_2265). The term "MamC" also includes functional fragments and variants of the protein derived from the *mamC* gene which may be expressed in biological systems or synthesized. Functional MamC fragments have been described previously (doi: 10.1016/j.jsb.2016.03.001; doi: 10.1107/S2059798317017491; patent WO 2017153996), in particular, functional fragments containing the MamM region-interaction region (MamC-MIL) (doi: 10.1016/j.jsb.2016.03.001). The functional fragments may be fused to another protein such as MBP. The term "MamC" also includes homologous proteins from other microorganisms (as nonexclusive examples: *Magnetococcales, Terasakiella, Magnetofaba, Magnetovibrio, Vibrio, Rhodospirillares, Magnetospirillum, Paramagnetospirillum, Bdellovibrionales, Bacteriovoracaceae, Desulfovibrio, Rhodobium, Desulfotignum, Mariprofundus* or other examples of *Alphaproteobacteria, Gammaproteobacteria, Deltaproteobacteria* or others) and/or variants of those proteins which may be expressed in biological systems or synthesized.

The term "Mms6" refers to a full-length protein which is derived from the *mms6* gene (NCBI Database, gene accession number ABK44766.1, protein accession Mmc1_2275). The term "Mms6" also includes functional fragments and variants of the protein derived from the *mms6* gene which may be expressed in biological systems or synthesized. The term "Mms6" also includes homologous proteins from other microorganisms as nonexclusive examples: *Magnetococcales, Terasakiella, Magnetofaba, Magnetovibrio, Vibrio, Rhodospirillares, Magnetospirillum, Paramagnetospirillum, Bdellovibrionales, Bacteriovoracaceae, Desulfovibrio, Rhodobium, Desulfotignum, Mariprofundus* or other examples of *Alphaproteobacteria, Gammaproteobacteria, Deltaproteobacteria* or others) and/or variants of those proteins which may be expressed in biological systems or synthesized.

The term "Mms7" refers to a full-length protein which is derived from the *mms7* gene (UniProtKB reference number Q2W8R9). The term "Mms7" also includes functional fragments and variants of the protein derived from the *mms7* gene which may be expressed in biological systems or synthesized. The term "Mms7" also includes homologous proteins from other microorganisms as nonexclusive examples: *Magnetococcales, Terasakiella, Magnetofaba, Magnetovibrio, Vibrio, Rhodospirillares, Magnetospirillum, Paramagnetospirillum, Bdellovibrionales, Bacteriovoracaceae, Desulfovibrio, Rhodobium, Desulfotignum, Mariprofundus* or other examples of *Alphaproteobacteria, Gammaproteobacteria, Deltaproteobacteria* or others) and/or variants of those proteins which may be expressed in biological systems or synthesized.

The terms "Gram-positive" and "Gram-negative" refer to two broad categories of bacteria based on their response to the Gram staining technique. This staining method was developed by Hans Christian Gram in the XIX century and is widely used to differentiate bacterial species into two groups based on their cell wall structure. The cell wall of Gram-positive bacteria has a thick layer of peptidoglycan. When subjected to the Gram stain, Gram-positive bacteria retain the crystal violet dye and appear purple or blue under a microscope. Typical Gram-positive bacteria are, for example, *Staphylococcus, Enterococcus, Streptococcus,* or *Bacillus,* among many others. On the other hand, the cell wall of Gram-negative bacteria has a thinner layer of peptidoglycan, in a periplasmic space and additionally, they have an outer membrane composed of lipopolysaccharides (LPS) and proteins. During the Gram stain procedure, the thin peptidoglycan layer is not sufficient to retain the crystal violet dye. Instead, decolored cells retain the pink counterstain. Examples of Gram-negative bacteria are *Escherichia coli, Salmonella* or *Pseudomonas,* among many others.

The classification into Gram-positive or Gram-negative has implications for bacterial susceptibility to certain antibiotics, as well as for understanding aspects of bacterial pathogenesis and physiology. But it is important to note that while the Gram stain could be a useful tool for the majority of bacteria, important exceptions and variations exist, and some bacteria may not neatly fit into these two categories (doi: 10.1111/mmi.14469), here referred with the term "other microorganisms outside Gram-positive or Gram-negative".

a) Families *Mycobacteriaceae* and *Nocardiaceae* (Order Mycobacterales), have a distinctive cell wall that does not conform to the typical Gram-positive or Gram-negative classification used for most bacteria, even though at phylogenetic level are classified as a Gram-positive. The cell wall of these microorganisms is characterized by a high lipid content, specifically mycolic acids (long-chain fatty acids that are linked to arabinogalactan, forming a complex structure), which contribute to its unique structure and properties. This complex cell wall structure is responsible for several features of these bacteria, including resistance to desiccation, resistance to conventional antibiotics and staining characteristics different to the Gram-positive or Gram-negative bacteria. These microorganisms were called acid-alcohol resistant (AAR) due to their particular ability to retain a red colorant after acid-alcohol decoloration that decolorates both Gram-positive and Gram-negative bacteria. The evolutionary origin of this type of cell wall remains a mystery (doi: 10.3389/fmicb.2018.02341). To this group belongs, for example, genera *Mycobacterium* and *Nocardia,* both with species of clinical interest.

b) *Mycoplasma* is a genus of bacteria that belongs to the class *Mollicutes.* Unlike the most of bacteria, mycoplasmas are characterized by the absence of a cell wall. This lack of a rigid cell wall makes them resistant to antibiotics that target cell wall synthesis, such as penicillin and other beta-lactam antibiotics. The absence of a cell wall in this group has implications for their physiology, pathogenicity, and interactions with the host. It also makes them susceptible to osmotic changes, which is why they are often found in environments with isotonic conditions, such as the mucous membranes of the respiratory and urogenital tracts. This genus includes species of clinical interest such as *Mycoplasma pneumoniae* (doi: 10.1007/s00284-022-03103-0). Additional examples of bacteria lacking a typical bacterial cell wall include *Chlamidiae* or *Tenericutes.*

The term "microbial biofilms" refers to structured associations of microorganisms connected by extracellular polymeric substances of different composition. This growth state favors the development of micro-niches that increase the metabolic heterogeneity and drastically contributes to (infection) persistence. Over 80% of microbial infections are due to biofilms. Antimicrobial resistance is several orders of magnitude higher against biofilm-embedded bacteria than against their planktonic counterparts due to the lower drug bioavailability caused by the extracellular matrix.

The term "intracellular pathogens" refers to (micro)organisms or entities that are capable of growing and reproducing inside host cells. They have evolved unique adaptations to modify host cellular processes that support key infection, promoting their survival, replication and dissemination. They could be facultative or obligate. Some examples are the viruses, bacteria like *Listeria monocytogenes, Staphylococcus aureus, Mycobacterium tuberculosis, Mycoplasma genitalium, Chlamydia, Neisseria, Rickettsia, Shigella, Brucella, Legionella pneumophila, Salmonella* spp, and protozoa like *Leishmania* and some stages of *Trypanosoma cruzi.* Facultative intracellular pathogens refer to (micro) organisms able to live and reproduce either inside or outside host cells while obligate intracellular pathogens cannot. Facultative intracellular pathogens can invade host cells to gain selective advantages.

## DESCRIPTION

BMNPs (Biomimetic magnetic nanoparticles) are magnetite nanoparticles of $39 \pm 7$ nm radius that mimic the magnetosome crystals synthesized by magnetotactic bacteria. They are produced *in vitro* by chemical precipitation in presence of MamC, a magnetosome-associated protein from *Magnetococcus marinus* MC-1. MamC controls the growth of magnetite crystals and allows to obtain nanoparticles with improved magnetic and surface characteristics. The presence of MamC in the external layers of the BMNPs provides them with functional groups to interact with molecules without needing post-production coatings, and an isoelectric point of 4.4, which means that they present negative net charge at physiological pH (Garcia-Rubia et al., 2018, doi: 10.1021/acs.langmuir.8b03109). The presence of MamC in the external layers allow the binding of BMNPs to molecules by mean of non-covalent interactions, mainly by electrostatic interactions or other non-covalent interaction such hydrophobic interaction or others. The advantage of forming nanoassemblies by this type of non-covalent interaction is that release is favored at acidic pH values, something crucial in clinical environments. The disadvantage is that much care should be taken to avoid the breakage of this interaction before the nanoformulation reaches the target, that could result in drug dissemination and/or an inefficient drug carrying.

The production of BMNPs is well known in the art as illustrated in EP3875433 or in Garcia-Rubia et al. 2018 (doi: 10.1021/acs.langmuir.8b03109). Nevertheless, just for illustration purposes, a non-limiting method of manufacturing BMNPs is illustrated as follows. In particular, a non-limiting method for producing a composition comprising a substantially pure mineral phase of superparamagnetic magnetite, comprises the following steps: (a) preparing a carbonate solution, (b) adding an $Fe^{3+}$ salt, preferably $FeCl_3$ to the carbonate solution, (c) adding MamC from a concentrated MamC stock and, optionally, Mms6 and Mms7 to the solution obtained in step (b), (d) incubating the solution obtained in step (c) for at least 30 minutes, (e) adding an $Fe^{2+}$ salt, preferably $Fe(ClO_4)_2$ to the solution obtained in step (d), and (f) adjusting the pH of the solution obtained in step (e) to pH 9 using a base; wherein, the method is performed at 25°C and 1 atmosphere of pressure, wherein the solutions used are previously deoxygenated, and the method is preferably performed under anoxic conditions, i.e., less than 100 ppb oxygen in the solution, most preferably, less than 40 ppb.

In connection to the present invention, and as indicated in example 1, the authors of the present invention have noticed that BMNPs, disaggregated under different protocols, behave differently, resulting in different efficiencies of the treatment. For instance, BMNPs from Group I (that underwent a specific disaggregation protocol) did not significantly alter cell viability after laser irradiation, as ~85% of tumoral HepG2 cells survived (this procedure would not be considered cytotoxic). On the contrary, BMNPs from Group II (that underwent a different disaggregation protocol) induced, following laser irradiation, a significant tumour cell death, as only ~46% of cells survived. In this case, the treatment was considered cytotoxic. This significantly higher cell death is related to the higher internalization percentage of Group II BMNPs. That is, a critical aspect for the efficiency of BMNPs as therapeutic agents is that BMNPs must be dispersed or disaggregated (not aggregated) prior and during the treatment of tumour cell lines, bacteria cultures, bacterial biofilms, intracellular pathogens and even prior use in biotechnological applications. The results illustrated herein, in the context of cancer treatment, show that aggregated instead of dispersed BMNPs are not internalized into the cells and, therefore, this results in the loss of efficiency of the treatment.

Therefore, a first aspect of the present invention refers to a composition comprising BMNPs (Biomimetic magnetic nanoparticles) or BMNP nanoassemblies, characterized in that said BMNPs are disaggregated or dispersed. Alternatively, the first aspect of the present invention refers to a composition comprising: (i) a substantially pure mineral phase of superparamagnetic magnetite, (ii) the MamC protein, and (iii) optionally, the Mms6 and/or Mms7 proteins; wherein, at least components (i) and (ii) form superparamagnetic magnetic nanoparticles containing up to 20 wt% of MamC and/or up to 20 wt% of Mms6 and/or up to 20 wt% of Mms7, most preferably containing 5 wt% of MamC and 0 wt% of Mms6 and Mms7, with a mean particle size between 5 and 120 nm, preferably between 30 and 50 nm, more preferably about 39 $\pm$ 7 nm, characterized in that said superparamagnetic magnetic nanoparticles containing MamC are disaggregated or dispersed.

As already indicated, an important step for using BMNPs and any nanoassemblies thereof is the disaggregation step. The output for not disaggregating BMNPs and any nanoassemblies thereof correctly or doing it by using an incorrect protocol could lead to a total loss of cytotoxicity and such, of the efficiency of the treatment. Aggregation leads to (among others): (1) difficulty to internalized cells, (2) loss of surface area of the nanoparticle ready to interact with the drug to form nanoassemblies, (3) loss of surface area of the nanoassembly ready to interact with the target (i.e, virus, prokaryote or eukaryote cell surface, target molecule), (4) defective drug releases, (5) impediment of rotation of nanoparticles/nano assemblies following application of an alternate magnetic field, resulting in failures of the magnetic hyperthermia and (6) shielding when a laser is applied to generate photothermia.

To correctly understand the importance of disaggregation, the following needs to be considered:

1. Both BMNPs and any nanoassemblies thereof are charged at physiological pH, which helps disaggregation by electrostatic repulsions.
2. Both BMNPs and any nanoassemblies thereof tend to aggregate by magnetic dipole interaction.
3. Thermal energy favors disaggregation.
4. Temperature increases above a certain threshold may break the electrostatic (or other non-covalent) bonding between a therapeutic molecule or drug and BMNPs in the nanoassemblies.
5. Short distances among nanoparticles or among nanoassemblies favor magnetic dipole interaction, thus prompting aggregation.
6. Disaggregation depends on the environment of the treatment (composition of the matrix, density)

In the present invention, a particularly useful disaggregation method or step, prior to using the BMNPs, comprises agitation of a BMNP dispersion of an adequate concentration that avoids shorting distances among BMNPs to prevent magnetic dipole interaction, while increases thermal energy to favor electrostatic repulsion among BMNPs. With these goals, the protocol includes working with BMNPs dispersions with a concentration of < 100 mg/mL, preferably about 15 mg/mL, subjected to a first cycle of vortex of about 1 second to 30 minutes at 30 to about 9000 rpm, preferably 30 seconds at about 3000 rpm, optionally followed by a sonication step, such as by using sonicator bath for 2-40 minutes at 10-100W, preferably 5 minutes at 35W, and preferably followed by a second cycle of vortex of about 1 second to 30 minutes at 30 to about 9000 rpm, preferably 30 seconds at about 3000 rpm. In the present invention, the term "about" or the term "~ " is +/- 10% of the indicated value. Preferably the disaggregation step, in particular the cycles of vortex and the sonication step, if any, are carried out at a temperature below 37°C, preferably below 35°C, more preferably below 30°C, but preferably above about 4°C.

In the present invention, a particularly useful disaggregation method or step, prior to using the BMNPs nanoassemblies, comprises the use of a nanoassembly dispersion of an adequate concentration that: (1) avoids shorting distances among nanoassemblies to prevent magnetic dipole interaction, (2) increases the thermal energy to favor electrostatic repulsion among nanoassemblies while (3) keeps the temperature below 30°C to prevent the breakage of the non-covalent (preferably electrostatic) bond that maintains the chemotherapeutic agent bond to BMNPs. With these goals, the protocol includes working with nanoassemblies dispersions, at a temperature below 37°C, preferably below 35°C, more preferably below 30°C, to prevent the breakage of the non-covalent bond, with a concentration of < 100 mg/mL, preferably about 15 mg/mL, subjected to a first cycle of vortex of about 1 second to 30 minutes at 30 to about 9000 rpm, preferably 30 seconds at about 3000 rpm, preferably followed by a second or subsequent cycle of vortex of about 1 second to 30 minutes at 30 to about 9000 rpm, preferably 30 seconds at about 3000 rpm. It is important to avoid sonication, as it can increase the temperature above 30°C and induce the release of the molecule previously attached to BMNPs. It is important not to increase vortex time, as this may lead to sample heating and, consequently, to drug release from the nanoassemblies. Preferably the disaggregation step, in particular the cycles of vortex are carried out at a temperature below 37°C, preferably below 35°C, more preferably below 30°C, but preferably above about 4°C.

It is noted that the BMNPs or BMNP nanoassemblies can go to one or more disaggregation cycles, as indicated above, in the same or different media. In this sense, it is noted that the use of AS-48_BMNPs (or, in general molecule-BMNPs and/or BMNPs) for different treatment requires a previous disaggregation of the nanoparticles/nanoassemblies in the relevant environments. Usually these environments (culture media) contain nutrients (including sugar, peptides, proteins, amino acids), that may lead to nanoparticle/nanoformulation aggregation due to different interactions of these nanoparticles/nanoformulations with these molecules. Once disaggregated in these media, then they can be added to the relevant culture and disaggregated again.

As used herein, a BMNP nanoassembly is understood as the result of binding to the surface of the BMNPs by non-covalent interactions, mainly electrostatic interactions or other weaker interactions such as hydrophobic or others, therapeutic agents exposing functional charged groups, such as the cationic peptide AS-48, or hydrophobic regions. Useful therapeutic agents in the present invention can be selected and combined from any one from the list consisting of: Actinomycin. All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine and Vinorelbine. Antibiotics: aminoglycosides (e.g. gentamicin, amikacin, tobramycin, plazomicin), carbapenems (e.g. imipenem, meropenem), cephalosporins (e.g. cephalexin, cefdinir, ceftazidime, cefepime), fluoroquinolones (e.g. nalidixic acid, ciprofloxacin, levofloxacin), glycopeptides (e.g. vancomycin, teicoplanin), lipopeptides (e.g. daptomycin), polymyxins (e.g. colistin, polymyxin B), macrolides (e.g. erythromycin, clarithromycin, azithromycin), monobactams (e.g. aztreonam, nocardicin A), oxazolidinones (e.g. linezolid, tedizolid), penicillins (e.g. amoxicillin, piperacillin, oxacillin), rifamycins (e.g. rifampicin, rifabutin, rifamixin), sulfonamides (e.g. sulfadiazine, sulfamethoxazole), pyrimidines (e.g. trimethoprim, pyrimethamine), streptogramins (e.g. pristinamycin, dalfopristin, quinupristin), tetracyclines (e.g. doxycyline, tigecycline), chloramphenicol, clindamycin and lincomicins, fosfomycin, pleuromutilins (e.g. lefamulin, valnemulin), metronidazole, mupirocin, nitrofurantoin, ethambutol. Antimicrobial peptides: e.g. defensins, cathelicidins, indolicidins, polyphemusins, insect antimicrobial peptides (apidaecins, cecropins, mellitins, proline-rich antimicrobial peptides, protegrins and arginine-rich antimicrobial peptides), plant antimicrobial peptides (thionins, defensins, amphibians and other animal derived antimicrobial peptides (magainins brevinins, ranateurins, bombinins, esculentins, rugosins, temporins, citropins), ribosomally produced and posttranslationally modified peptides (RiPPs; circular bacteriocins such as AS-48 and/or its derivatives, cyanobactins, bottromycins, lasso-peptides, sactipeptides among others), bacteriocins, non-ribosomally synthesized peptides (gramicidins, polymyxins, tyrocidines, daptomycin). Peptidomimetics (e.g. omiganan, lytixar, brilacidin, pexiganan), synthetic antimicrobial peptides (novispririn, iseganan, RR, HB1345, RRIKA, DASamP1, HHC-10, HHC-36), peptoids/ampetoids (ampetoid 1, GN-2, GN4, peptoid 13, L3, L4, L6, C3, C4, C6) and peptaibols (emerimicin, trichokonins, chilenopeptins, septocylindrins, hyporientalin, trichorzianines, pentadecaibins, leucinostatin). Antimicrobial inorganic salts (e.g. copper and silver salts, among other). Antiseptics such as quaternary ammonium salts (benzalkonium chloride, cetyltrimethylammonium chloride), boric acid, chlorhexidine. Exopolysaccharide-degrading enzymes (such as glucanohydrolases and dispersin B, among others). Quorum sensing Inhibitors (QSI).

Moreover, in connection to the disaggregation method described herein, it is further noted that:

1) The BMNPs or BMNP nanoassemblies' stock should not be lyophilized (to avoid concentrating the suspension and shorting distances among nanoparticles or nanoassemblies).
2) The BMNPs or BMNP nanoassemblies stock should not be frozen (to maintain thermal energy). In fact, the BMNP nanoassemblies should not be cooled below 4°C, and, preferably, they should be stored at room temperature.
3) The BMNPs or BMNP nanoassemblies should be collected from the stock by using a magnet. They should not be centrifuged.

As regards the superparamagnetic magnetic nanoparticles containing MamC described herein, a preferred embodiment of the first aspect of the invention indicates that the superparamagnetic magnetic nanoparticles containing MamC (BMNPs) are composed of 80-95 wt% of magnetite and 5-20 wt% of MamC and/or 0-20 wt% of Mms6 and/or 0-20 wt% of Mms7, more preferably BMNPs are composed of ~95 wt% of magnetite and ~5 wt% of MamC. The isoelectric point could be in the range 3 to 7, most preferably is of ~4.4.

In another preferred embodiment of the first aspect of the invention, in the superparamagnetic magnetic nanoparticles containing MamC (BMNPs), there are no detectable levels of siderite in the composition. In an even more preferred manner, the levels of goethite are $\leq$ 10% of the total solid.

In another preferred embodiment of the first aspect of the invention, the mean particle size of the superparamagnetic magnetic nanoparticles containing MamC (BMNPs) is 30-50 nm. Preferably, the mean particle size of the magnetic nanoparticles is 30-40 nm, more preferably about $39\pm7$ nm. It is herein noted that the mean particle size is determined using transmission electron microscopy. In the examples of the present invention, the sizes were obtained by measuring the size of more than 1000 crystals in each transmission electron microscopy image.

In another preferred embodiment of the first aspect of the invention, the wt% of MamC in the superparamagnetic magnetic nanoparticles containing MamC (BMNPs) is 2-20 wt%. Preferably, the wt% of MamC in the BMNPs is 2-10 wt%. In the best conditions, the wt% of MamC in the BMNPs is determined by thermogravimetric analysis. In the examples of the present invention, the wt% of MamC in the BMNPs is 5 wt%.

It is further noted that in another preferred embodiment of the first aspect of the invention, the superparamagnetic magnetic nanoparticles containing MamC (BMNPs) may further comprise other proteins involved in the formation of magnetite in the magnetosomes of bacteria and/or other proteins with acidic domains capable of binding iron and/or those with such a structure that could provide a template for magnetite nucleation and growth. Non-limiting examples of such proteins include Mms6, Mms7, MmsF/MamF and their homologous proteins in different magnetotactic bacteria. In the best conditions, the composition further comprises Mms7.

In another preferred embodiment of the first aspect of the invention, the isoelectric point of the superparamagnetic magnetic nanoparticles containing MamC (BMNPs) is 3-7. Preferably, the isoelectric point of the BMNPs is 3-5. In yet another preferred embodiment of the first aspect of the invention, the isoelectric point of the BMNPs is calculated from measurements of the electrophoretic mobility. In the examples of the present invention, the isoelectric point of the BMNPs is 4.4.

The nanoparticles formed using the MamC protein exhibit a large magnetization per particle at room temperature that is equal to or higher than that of the nanoparticles obtained through inorganic methods or through the use of Mms6 protein alone. Thus, in another preferred embodiment of the first aspect of the invention, the magnetization of the BMNPs is 40-70 emu/g at 300K when an external magnetic field of 50K Oe is applied. Preferably, the magnetization of the BMNPs is 55-65 emu/g at 300K when an external magnetic field of 50K Oe is applied. In optimal conditions, the magnetization of the BMNPs is 55 emu/g (61 emu/g when the amount of MamC in the crystal is taken into account) at 300K when an external magnetic field of 50K Oe is applied.

In another preferred embodiment of the first aspect of the invention and as already indicated, the superparamagnetic magnetic nanoparticles containing MamC (BMNPs) form BMNPs nanoassemblies. As indicated, a BMNP nanoassembly is understood as the result of linking to the surface of the BMNPs by non-covalent bond, mainly electrostatic interactions or other weaker interactions such as hydrophobic or others, therapeutic agents exposing functional charged groups, such as the cationic peptide AS-48, and/or hydrophobic regions. The therapeutic agent can be any agent which has a therapeutic effect when administered in a therapeutically effective amount.

In another preferred embodiment of the first aspect of the invention, the composition consists of: (i) a substantially pure mineral phase of superparamagnetic magnetite, (ii) MamC, and (iii) optionally, Mms6 and/or Mms7; wherein, at least components (i) and (ii) form superparamagnetic magnetic nanoparticles containing up to 20 wt% of MamC, up to 20 wt% of Mms6 and up to 20 wt% of Mms7. In tme most preferred embodiment the superparamagnetic nanoparticles contain up to 5 wt% of MamC (MamC-mediated BMNPs are thus composed of ~95 wt% of magnetite and ~5 wt% of MamC), with a mean particle size between 30 and 120 nm, an isoelectric point between 5-7, most preferably of ~4.4.

It is noted that, preferably, the composition described throughout the first aspect of the present invention is not derived, obtained or obtainable from a magnetosome. More preferably, the composition is not obtained from a magnetosome. To clarify, in this condition, while the proteins used in the composition may be derived from a magnetosome, the BMNPs are preferably obtained using the *in vitro* precipitation approach disclosed in the present invention. The term "magnetosome" refers to both natural magnetosomes present in magnetotactic bacteria as well as recombinant magnetosomes or magnetosome-like structures that are produced by a host which does not normally contain magnetosomes or magnetosome-like structures. In particular, the approach described herein comprises the following steps: (a) preparing a carbonate solution, (b) adding an $Fe^{3+}$ salt, preferably $FeCl_3$, to the carbonate solution, (c) adding MamC and, optionally, Mms6 and/or Mms7 from concentrated stocks to the solution obtained in step (b), (d) incubating the solution obtained in step (c) for at least 30 minutes, (e) adding an $Fe^{2+}$ salt, preferably $Fe(ClO_4)_2$, to the solution obtained in step (d), and (f) adjusting the pH of the solution obtained in step (e) to pH 9 using a base; wherein, the method is performed at 25°C and 1 atmosphere of pressure, wherein the solutions used are previously deoxygenated, and the method is preferably performed under anoxic conditions, i.e., less than 100 ppb oxygen, mot preferably less than 40 ppb oxygen in the solution. This method is described in EP3875433.

The composition described in the first or second aspect (please see below) of the invention, once disaggregated or dispersed as indicated herein, is from herein after referred to as the composition of the present invention.

Although the composition of the present invention can be produced for any suitable method, in a second aspect of the present invention, the BMNPs of the composition of the present invention are obtained, obtainable or prepared by a method comprising the following steps:

    a) preparing a carbonate solution;
    b) adding an $Fe^{3+}$ salt, preferably $FeCl_3$ to the carbonate solution;

c) adding MamC and, optionally, Mms6 and/or Mms7 from a concentrated stock (at least of 1 mg/mL, preferably of 2 mg/mL) to the solution obtained in step (b);

d) incubating the solution obtained in step (c) for at least 30 minutes;

e) adding an $Fe^{2+}$ salt, preferably $Fe(ClO_4)_2$ to the solution obtained in step (d); and

f) adjusting the pH of the solution obtained in step (e) to an alkaline pH, preferably 9 or higher, more preferably 9;

wherein the method is preferably performed at 25°C and 1 atmosphere of pressure and all the solutions used are previously deoxygenated; and wherein the composition is preferably further characterized by comprising, after several cycles of thorough wash with oxygen-free MilliQ water following magnetic concentration of the solids, no detectable levels of siderite as measured by X-ray diffraction and a goethite content not exceeding 5 wt%.

Disaggregation (non-aggregation) of BMNPs is crucial to ensure efficiency. It is important to notice that being BMNPs superparamagnetic and charged at physiological pH values, BMNPs tend to aggregate by magnetic dipole interaction and charge helps disaggregation by electrostatic repulsions, favored to a certain level by thermal energy. Therefore, it is important not to concentrate BMNP suspension and/or to apply methods that favor this concentration (lyophilization, centrifugation as examples, but not exclusive), while favoring thermal energy, thus avoiding excessive refrigeration and/or freezing that could favor magnetic dipole interaction among BMNPs.

Therefore, in a preferred embodiment, the method is further characterized by having a disaggregation step. In particular, the disaggregated or dispersed superparamagnetic magnetic nanoparticles comprising MamC are obtained by subjecting a suspension or dispersion comprising the superparamagnetic magnetic nanoparticles directly obtained by the above method at a concentration of less than 100 mg/mL, preferably about 15 mg/mL, to a first cycle of vortex of about 1 second to 30 minutes at 30 to about 9000 rpm, preferably 30 seconds at about 3000 rpm, optionally followed by a sonication step, and followed by a second or subsequent cycle of vortex of about 1 second to 30 minutes at 30 to about 9000 rpm, preferably 30 seconds at about 3000 rpm. These conditions have been clearly explained before and also applied to this specific embodiment. Preferably the disaggregation step, in particular the cycles of vortex and the sonication step, if any, are carried out at a temperature below 37°C, preferably below 35°C, more preferably below 30°C, but preferably above about 4°C.

In a preferred embodiment of the second aspect of the invention, the method further comprises the step of functionalizing or binding the magnetic biomimetic nanoparticles with a therapeutic agent by non-covalent bond, mainly electrostatic interaction or other interactions such as hydrophobic ones, to the surface of the BMNPs. Therapeutic agents are preferably antibacterial agents, such as the cationic peptide AS-48 and/or any other antimicrobial, antiparasite and/or antitumoral agent. Useful therapeutic agents in the present invention can be selected from any one from the list consisting of: Actinomycin. All-trans retinoic acid, Azacitidine, Azathioprine, Bleomycin, Bortezomib, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Mechlorethamine, Mercaptopurine, Methotrexate, Mitoxantrone, Oxaliplatin, Paclitaxel, Pemetrexed, Teniposide, Tioguanine, Topotecan, Valrubicin, Vemurafenib, Vinblastine, Vincristine, Vindesine and Vinorelbine. Antibiotics: aminoglycosides (e.g. gentamicin, amikacin, tobramycin, plazomicin), carbapenems (e.g. imipenem, meropenem), cephalosporins (e.g. cephalexin, cefdinir, ceftazidime, cefepime), fluoroquinolones (e.g. nalidixic acid, ciprofloxacin, levofloxacin), glycopeptides (e.g. vancomycin, teicoplanin), lipopeptides (e.g. daptomycin), polymyxins (e.g. colistin, polymyxin B), macrolides (e.g. erythromycin, clarithromycin, azithromycin), monobactams (e.g. aztreonam, nocardicin A), oxazolidinones (e.g. linezolid, tedizolid), penicillins (e.g. amoxicillin, piperacillin, oxacillin), rifamycins (e.g. rifampicin, rifabutin, rifamixin), sulfonamides (e.g. sulfadiazine, sulfamethoxazole), pyrimidines (e.g. trimethoprim, pyrimethamine), streptogramins (e.g. pristinamycin, dalfopristin, quinupristin), tetracyclines (e.g. doxycyline, tigecycline), chloramphenicol, clindamycin and lincomicins, fosfomycin, pleuromutilins (e.g. lefamulin, valnemulin), metronidazole, mupirocin, nitrofurantoin, ethambutol. Antimicrobial peptides: e.g. defensins, cathelicidins, indolicidins, polyphemusins, insect antimicrobial peptides (apidaecins, cecropins, mellitins, proline-rich antimicrobial peptides, protegrins and arginine-rich antimicrobial peptides), plant antimicrobial peptides (thionins, defensins, amphibians and other animal derived antimicrobial peptides (magainins brevinins, ranateurins, bombinins, esculentins, rugosins, temporins, citropins), ribosomally produced and posttranslationally modified peptides (RiPPs; circular bacteriocins such as AS-48 or its derivatives, cyanobactins, bottromycins, lassopeptides, sactipeptides among others), bacteriocins, non-ribosomally synthesized peptides (gramicidins, polymyxins, tyrocidines, daptomycin). Peptidomimetics (e.g. omiganan, lytixar, brilacidin, pexiganan), synthetic antimicrobial peptides (novispririn, iseganan, RR, HB1345, RRIKA, DASamP1, HHC-10, HHC-36), peptoids/ampetoids (ampetoid 1, GN-2, GN4, peptoid 13, L3, L4, L6, C3, C4, C6) and peptaibols (emerimicin, trichokonins, chilenopeptins, septocylindrins, hyporientalin, trichorzianines, pentadecaibins, leucinostatin). Antimicrobial inorganic salts (e.g. copper and silver salts, among other). Antiseptics such as quaternary ammonium salts (benzalkonium chloride, cetyltrimethylammonium chloride), boric acid, chlorhexidine. Exopolysaccharide-degrading enzymes (such as glucanohydrolases and dispersin B, among others). Quorum sensing Inhibitors (QSI).

Therefore, in another preferred embodiment related to the magnetic biomimetic nanoparticles with a therapeutic agent indicated above (the BMNPs nanoassemblies), the method is further characterized by having a disaggregation step. In particular, the disaggregated or dispersed superparamagnetic magnetic nanoparticles comprising MamC are obtained by subjecting a solution or dispersion comprising the superparamagnetic magnetic nanoparticles directly obtained by the above method at a concentration of less than 100 mg/mL, preferably about 15 mg/mL, to a first cycle of vortex, at a temperature below 37°C, preferably below 35°C, more preferably below 30°C, of about 1 second to 30 minutes at 30 to about 9000 rpm, preferably about 30 seconds at about 3000 rpm, preferably followed by a second or subsequent cycle of vortex, at a temperature below 37°C, preferably below 35°C, more preferably below 30°C, of about 1 second to 30 minutes at 30 to about 9000 rpm, preferably about 30 seconds at about 3000 rpm. Again, these conditions have been clearly explained before and also applied to this specific embodiment. Disaggregation (non-aggregation) of the nanoassemblies is crucial to ensure efficiency.

Preferably the disaggregation step, in particular the cycles of vortex and the sonication step, if any, are carried out at a temperature below 37°C, preferably below 35°C, more preferably below 30°C, but preferably above about 4°C. In this sense, it is important to notice that being BMNPs superparamagnetic and being the nanoassemblies charged at physiological pH values, the nanoassemblies tend to aggregate by magnetic dipole interaction and charge helps disaggregation by electrostatic repulsions, favored to a certain level by thermal energy. It is important to note that thermal energy also breaks non-covalent bonds (as for example electrostatic or hydrophobic bonds). Therefore, temperature rises should be carefully controlled to avoid the breakage of the nanoassemblies while temperature decreases should also be avoided to prevent magnetic dipole interaction between the nanoassemblies. Moreover, concentrating the nanoassemblies will also favor magnetic dipole interaction, so the nanoassemblies concentration of the suspension has to be carefully controlled, further avoiding methods such as lyophilization, centrifugation or any other method that increase the concentration of the nanoassemblies and favor aggregation.

It is further noted that the present invention does not only explicitly refer, within the scope of the present invention, to the composition of the present invention as obtained or produced in the second aspect of the present invention, but also to the method indicated in this second aspect of the invention.

A third aspect of the present invention refers to the composition of the present invention embedded in PLGA or in one or more variants of poly[lactic-co-glycolic acid] (PLGA) or liposomes of different compositions and structures. It is noted that the further embedding of AS-48_BMNPs into PLGA significantly improves cell internalization of the nanoassembly.

On another noted, as illustrated in examples 2 and 3, the composition of the present invention or the composition of the third aspect of the present invention, preferably in the form of BMNPs nanoassemblies, can be used in therapy, preferably for the treatment of infections caused by microorganisms (including Gram-positive and Gram-negative or other not included in this group). This composition can also be used in diseases like cancer or those caused by other etiologic agents, such as viruses and parasites.

Therefore, a fourth aspect of the present invention refers to the composition of the present invention or the composition of the third aspect of the present invention for use in therapy.

A fifth aspect of the present invention refers to the composition of the present invention or the composition of the third aspect of the present invention, preferably in the form of BMNPs nanoassemblies, for used in the treatment of diseases, like cancer or infections caused by microorganisms (including Gram-positive and Gram-negative or other microorganisms outside Gram-positive or Gram-negative) or other etiologic agents, such as viruses and parasites. Typical Gram-positive bacteria included in the present invention are, for example, *Staphylococcus, Enterococcus, Streptococcus,* or *Bacillus,* among many others. Examples of Gram-negative bacteria included in the present invention are *Escherichia coli, Klebsiella, Salmonella* or *Pseudomonas,* among many others.

In particular, from example 2, we can conclude the following:

- Bacteriocin AS-48 immobilized on BMNPs inhibits growth of *M. tuberculosis* in both extracellular and intracellular conditions, making it possible a potential local and directed treatment.
- AS-48_BMNP is efficient for killing intracellular *M. tuberculosis.*
- PLGA[AS-48_BMNP] nanoformulations were inactive against cultures of *extracellular M. tuberculosis,* but were active against *M. tuberculosis* residing in macrophages.
- Immobilization of bacteriocin AS-48 with BMNPs, with or without PLGA covering, is a promising approach to target intracellular pathogens such as *M. tuberculosis*
- Combination of nanoformulations containing bacteriocin AS-48 with subinhibitory concentrations of ethambutol does not affect antituberculosis activity of AS-48.

As used herein, the term "AS-48" shall be understood as a cationic and hydrophobic bacteriocin. The first isolated peptide is 70 amino acids long head-to-tail cyclized peptide produced by several *Enterococcus* species, with a potent bactericidal activity on Gram-positive bacteria. In general, Gram-negative bacteria show a naturally increased resistance to AS-48 due to the presence of the outer membrane in their wall cells. Due to N- and C-termini linkage, AS-48 is resistant to proteases and stable in a broad range of conditions (pH, temperature and salt concentrations). Bacteriocin AS-48 has been fully characterized from the genetical, structural, or biological activity points of view (Cebrián et al., 2023; doi: 10.3389/fmicb.2023.1110360). It's structure is public as pdb 1083 and 1E68. It is classified in literature as a model circular bacteriocin and a ribosomally produced and posttranslationally modified peptide (RiPP). Several mutants of AS-48 and truncations have been isolated which retain this antimicrobial activity and that should be referred to as AS-48 derivatives (doi: 10.3389/fmicb.2020.589666). It has received other names in literature such as eneroccocin EFS2, bacteriocin 21, enterocin 4 (doi: 10.1046/j.1365-2958.1998.01020.x). AS-48 has low toxicity against eukaryotic organisms and AS-48 and its derivatives display a potent activity against Gram-positive bacteria, some Gram-negative bacteria and eukaryotes from the family *Trypanosomatidae.*

Consequently, a sixth aspect of the present invention refers to the composition of the present invention or the composition of the third aspect of the present invention, preferably in the form of BMNPs nanoassemblies, for used in the treatment of intracellular pathogens. Preferably, the composition of the present invention or the composition of the third aspect of the present invention, preferably in the form of BMNPs nanoassemblies, is for used in the treatment of tuberculosis, that is, the disease caused by *M. tuberculosis.*

Furthermore, as shown in example 3, it is interesting to point out that (1) Synergy of the antibacterial treatment mediated by the nanoformulation AS-48_BMNPs with MH and PT was able to reduce the doses of AS-48 while keeping the efficiency of the treatment, and (2) The antibacterial effect was induced immediately after exposure to an AMF or to laser irradiation (in comparison, the time necessary to eradicate the infection at a MIC concentration is 6 days). These results open new opportunities for tuberculosis treatment and could be studied as well with other intracellular pathogens, especially when they are slow-growing bacteria that require long-lasting therapies.

Therefore, a seventh aspect of the present invention refers to the composition of the present invention or the composition of the third aspect of the present invention, for used in therapy in combination with magnetic hyperthermia (MH) and/or photothermia (PT) mediated by BMNP.

Preferably, the composition of the present invention or the composition of the third aspect of the present invention, is for used in the treatment of bacterial infections caused by Gram-positive and/or Gram-negative bacteria in combination with magnetic hyperthermia (MH) and/or photothermia (PT) mediated by BMNP.

Preferably, the composition of the present invention or the composition of the third aspect of the present invention, is for used in the treatment of intracellular pathogens in combination with magnetic hyperthermia (MH) and/or photothermia (PT) mediated by BMNP.

Preferably, the composition of the present invention or the composition of the third aspect of the present invention, is for used in the treatment of tuberculosis in combination with magnetic hyperthermia (MH) and/or photothermia (PT) mediated by BMNP. In this specific case, the use of BMNPs nanoassemblies by using AS-48 is particularly preferred.

More preferably, for any of the above embodiments of the seventh aspect of the present invention, the composition of the present invention or the composition of the third aspect of the present invention are subjected to alternating magnetic fields (AMF) during the treatment or during part of the treatment.

More preferably, for any of the above embodiments of the seventh aspect of the present invention, the composition of the present invention or the composition of the third aspect of the present invention are exposed to a laser in the near infrared (NIR) and transform the absorbed energy into heat during the treatment or during part of the treatment.

Furthermore, as illustrated in example 4, the results confirm that AS-48_BMNP nanoformulation is able to eradicate biofilms formed by Gram-positive bacteria such as *Enterococcus faecalis* and significantly reduce colony counts in biofilms formed by Gram-negative bacteria such as *Pseudomonas aeruginosa* in combination with hyperthermia.

Therefore, an eight aspect of the present invention refers to the composition of the present invention or the composition of the third aspect of the present invention, for used in the treatment of biofilms formed by Gram-positive or Gram-negative bacteria, preferably in combination with magnetic hyperthermia (MH) and/or photothermia (PT) mediated by BMNP.

Finally, it is noted that the present invention provides a pharmaceutical composition which comprises the composition of the present invention or the composition of the third aspect of the present invention and a pharmaceutically acceptable vehicle and/or diluent.

In a preferred embodiment of the invention, the pharmaceutical composition may comprise one or more dispersion(s) which are suitable for intravenous, intraarterial, intramuscular and/or subcutaneous administration. In another embodiment, the pharmaceutical composition may comprise one or more dispersion (s) which are suitable for sublingual, buccal and/or inhalation-mediated administration routes. In an alternative embodiment, the pharmaceutical composition may comprise one or more aerosol(s) which are suitable for inhalation-mediated administration.

In a preferred embodiment of the invention, the pharmaceutical composition may comprise one or more cream(s) and/or ointment(s) which are suitable for topical administration. In a preferred embodiment of the invention, the pharmaceutical composition may comprise one or more suppositories which are suitable for rectal or vaginal administration. In this embodiment, the composition may be used in order to achieve a loco-regional effect.

The following examples are merely for illustration purposes and should not limit the scope of the present invention.

**EXAMPLES**

**EXAMPLE 1**

Biomimetic magnetic nanoparticles (BMNPs) are a novel carrier system for therapeutic agents based on their surface and magnetic properties that allow magnetic concentration and prevent drug dissemination. BMNPs are biocompatible and also demonstrate the capacity to function as hyperthermia agents (both magnetic hyperthermia and photothermia).

A critical aspect for the efficiency of BMNPs as therapeutic agents is that BMNPs must be dispersed (not aggregated) prior and during the treatment of tumour cell lines. The results illustrated herein show that aggregated instead of dispersed BMNPs are not internalized into the cells and, therefore, this results in the loss of efficiency of the treatment.

## 1.1 MATERIALS AND METHODS

### 1.1.1 Disaggregation of BMNPs (Biomimetic magnetic nanoparticles)

Group I: Before treating cells, BMNPs were vortexed for 30 seconds at 3000 rpm.

Group II: **Before treating cells with BMNPs, to ensure BMNPs dispersion, BMNPs were vortexed for 30 seconds at 3000 rpm, then underwent a sonication cycle of 5 min (35W) and then vortexed again for 30 seconds.**

### 1.1.2. Internalization of BMNPs

The incorporation of BMNPs into human hepatoblastoma HepG2 cells was evaluated using the potassium thiocyanate method as follows. Cells were seeded in 12-well plates (300,000 cells per well) and treated with BMNPs (both from Group I and from Group II) at 300 $\mu$g/mL in MEM/10% FBS for 24 hours. Afterward, non-internalized BMNPs were washed away 3 times with PBS, and cells were trypsinized and transferred to 2 mL tubes. Each tube was centrifuged at 1,000 rpm for 5 minutes. Subsequently, to dissolve the cell pellet, 37% HCl/10% HzOz was added and maintained for 20 minutes at room temperature. Immediately, one millilitre of 1% potassium thiocyanate in Milli-Q water was added to the tubes. As a final step, absorbance at 490 nm was measured, and a standard calibration curve was used to obtain the endogenous iron content of the cells.

### 1.1.3. Visualization of cells treated with BMNPs

The appearance of cells incubated with BMNPs (both from Group I and from Group II) was analysed using inverted optical microscopy.

### 1.1.4. Cell viability

The antiproliferative effect induced by BMNPs (both from Group I and from Group II) in combination with hyperthermia (magnetic hyperthermia and photothermia) was evaluated in HepG2 cells. Initially, cells were seeded onto 12-well plates at 300,000 cells per well. Then, cells were incubated for 24 hours at 37 °C with BMNPs from Group I (300 $\mu$g/mL). An identical protocol was followed to treat HepG2 cells with BMNPs from Group II (300 $\mu$g/mL). After the 24-hours treatment, non-internalized BMNPs were removed in both series of experiments, and the cells (containing internalized BMNPs) were irradiated for 600 s from the top with the NIR (near InfraRed, A = 808 nm) laser set of 0.25 W/cm$^2$. After the treatments, cell viability for the two series of experiments was assessed with 1 mM resazurin in PBS. Fluorescence intensity, an indicator of cellular activity, was measured at $\lambda$ex = 535/Aem = 590 nm using a microplate reader.

## 1.2. RESULTS

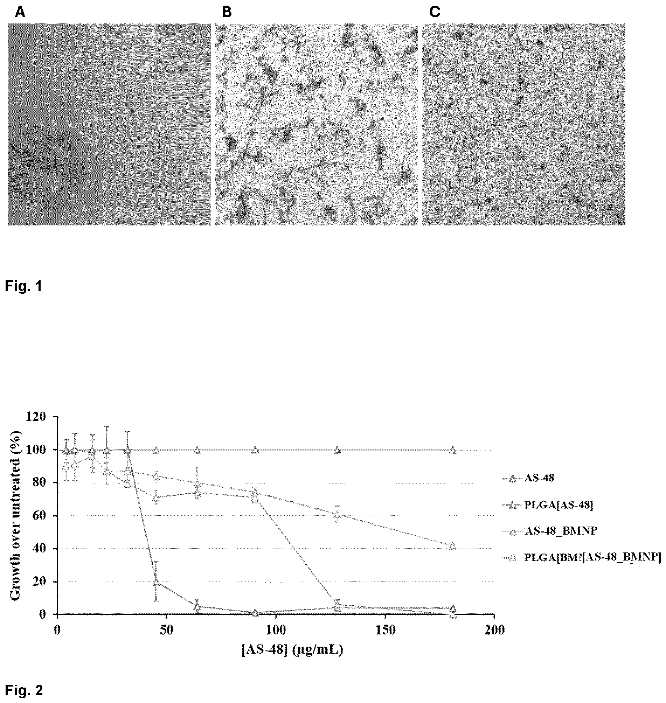

The results are provided in figure 1; in particular figure 1A shows HepG2 cells non treated (without BMNPs), figure 1B shows HepG2 cells incubated with BMNPs from Group I, and figure 1C incubated with BMNPs from Group II. As it can be seen, BMNPs from Group I are much more aggregated than those from Group II. In terms of the effect of this aggregation on the efficiency of the treatment, data are shown in Table 1 below.

**Table 1.** Data of internalization rate of BMNPs and percentage of cell viability (data from triplicates)

| Experiment type | Internalized [Fe] (mM) | % internalization | % cell viability before laser exposure | % cell viability after laser exposure |
|---|---|---|---|---|
| Group I | 0.494 | 13 | 100.0 ± 2.8 | 85.6 ± 2.5 |
| Group II | 1.44 | 38 | | 46.1 ± 0.3 |

BMNPs from Group I had a much lower percentage of internalization (13%) than those from Group II (38%). This internalization difference did not have an impact on the cytocompatibility of BMNPs from any of the two groups before laser irradiation. Yet, it had a considerable impact in tumour cell death following laser exposure. BMNPs from Group I did not significantly alter cell viability after laser irradiation, as ~85% of tumoral HepG2 cells survived (this procedure would not be considered cytotoxic). On the contrary, BMNPs from Group II induced, following laser irradiation, a significant tumour cell death, as only ~46% of cells survived. In this case, the treatment was considered cytotoxic. This significantly higher cell death is related to the higher internalization percentage of Group II BMNPs.

**EXAMPLE 2 EFFICIENCY OF BMNP MEDIATED NANOFORMULATIONS AGAINST *Mycobacterium tuberculosis.***

## 2.1. MATERIALS & METHODS

### 2.1.1. Bacterial strains and growth conditions

*M. tuberculosis* H37Rv (ATCC 25618) and *M. tuberculosis* H37Rv-Luc (a derivative of *M. tuberculosis* H37Rv ATCC 25618 strain constitutively expressing the firefly luciferase luc gene from *Photinus pyralis* cloned into pMV306hsp integrative vector [Andreu N, Zelmer A, FletcherT, Elkington PT, Ward TH, Ripoll J, Parish T, Bancroft GJ, Schaible U, Robertson BD, Wiles S. Optimisation of bioluminescent reporters for use with mycobacteria. PLoS One. 2010 May 24;5(5):e10777. doi: 10.1371/journal.pone.00107777] were routinely cultured at 37 °C in Middlebrook 7H9 broth (Difco) supplemented with 10% Middlebrook Albumin-Dextrose-Catalase (ADC; Difco) and 0.05% Tween 80. Kanamycin (Sigma-Aldrich) at 20 $\mu$g/mL was added to the medium when propagating *M. tuberculosis* H37Rv-Luc.

### 2.1.2 Drugs, chemicals and nanoparticle samples

Resazurin and antituberculosis drugs (ethambutol) were purchased from Sigma-Aldrich. Antimicrobial bacteriocin AS-48 was purified from *Enterococcus faecalis* UGRA10 strain as described in Cebrian et al. 2015 (doi: 10.1016/j.jsb.2015.03.006). Biomimetic magnetic nanoparticles (BMNPs) associated to bacteriocin AS-48, with or without polylactic-co-glycolic acid (PLGA) covering were produced and characterised according to Gaglio et al. 2022 (doi: 10.3390/pharmaceutics14122744). Prior to the following assays, the BMNPs or the AS-48_BMNP nanoassemblies were resuspended in the same culture medium as the cells and vortexed for 30 seconds (3000 rpm). To further disaggregate them, BMNPs were also sonicated for 10 min (35 W). AS-48_BMNP nanoassemblies were vortexed instead for additional 10 seconds (3000 rpm). The nanoassemblies could not be sonicated, in order to prevent the release of AS-48 from the surface of BMNPs.
All samples were tested in triplicate in at least two independent experiments.

### 2.1.3 Drug susceptibility assays (Minimal Inhibitory Concentration; MIC) on extracellularly growing bacteria

MICs of compounds and nanoparticle samples against cultures of *M. tuberculosis* strains were determined by broth microdilution method in Middlebrook 7H9 broth supplemented with 10% ADC and 0.2% glycerol (instead of Tween 80). Antimicrobial stocks (compounds and nanoparticle simples) at twice the maximal test concentration were prepared and 2-fold or 1 .4-fold serial dilutions of compounds and/or nanoparticle samples were performed in 75 $\mu$l of culture medium in clear 96-well flat-bottom plates. As a reference, bacteriocin AS-48 and nanoformulations containing AS-48 (i.e. AS-48_BMNP, and PLGA[AS-48_BMNP]) were tested in a range of concentrations from 256 to 4 $\mu$g/mL of bacteriocin AS-48; ranges of concentrations of BMNPs, PLGA, and PLGA[BMNP] were adjusted to match those concentrations present in the nanoformulations containing bacteriocin AS-48. Each well was inoculated with 75 $\mu$l of a fresh culture of *M. tuberculosis* H37Rv ATCC 25618 at a bacterial density of $2 \times 10^5$ CFU/mL. Positive and negative growth controls were included in at least three wells on each plate; wells containing positive growth controls consisted on *M. tuberculosis* H37Rv ATCC 25618 without further addition of any compound; negative growth controls consisted on Middlebrook 7H9 broth without bacterial inoculum. Additionally, the MIC of antituberculosis drug isoniazid was carried out in triplicate, by serially

diluting a 2 μg/mL stock solution of this drug in culture medium, as above.

Plates were incubated for 6 days at 37 °C. Then, plates were placed over a magnet in order to retain BMNPs at the bottom of the wells; supernatants from all wells were carefully transferred to a new 96-well plate before the addition of 30 μL of the bacterial growth indicator resazurin at 0.1 mg/mL. After 48 h of incubation, colour changes from blue (no growth) to pink (growth) were visually evaluated. Additionally, fluorescence of wells was measured at 530- and 590-nm excitation and emission wavelengths, respectively, in a Synergy HT microplate reader (BioTek); positive controls represented 100% growth and negative controls were taken as 0% growth. Absorbance of individual wells ($Abs_{Sample}$) was normalized to the percentage of growth (% $Growth_{Sample}$) using the absorbance of positive and negative growth controls (Abs 100% Growth control and Abs 0% Growth control, respectively) and the following formula:

$$\% \ Growth_{Sample} = \frac{Abs_{Sample} - Abs_{0\% \ Growth \ control}}{Abs_{100\% \ Growth \ control} - Abs_{0\% \ Growth \ control}} \ x \ 100$$

The lowest drug concentration that inhibited 90% growth was used to define the MIC value of each compound.

### 2.1.4 MIC against M. tuberculosis H37Rv-Luc infected macrophages.

*M. tuberculosis* H37Rv-Luc cultures grown to mid-exponential phase ($OD_{600}$ = 0.5-0.8) were pelleted, dispersed by using glass beads and resuspended in cell culture medium without antibiotics. Then, the supernatant was collected and centrifuged at 750 rpm for 5 min to pellet bacterial clumps. The upper part of the supernatant, containing a suspension of single bacterial cells, was recovered and the $OD_{600}$ measured in order to estimate the number of CFU/mL (an $OD_{600}$ of 0.125 corresponds to $10^7$ CFU/mL) before being used for infecting THP-1 cells.

Frozen stocks of human monocytic THP1 cells (ECACC 88081201) were thawed in RPMI-1640 medium with HEPES modification (Gibco) supplemented with 1% GlutaMAX (Gibco), 10% heat-inactivated fetal bovine serum (FBS; Gibco) and 1 mM sodium pyruvate (Sigma). Cells were maintained with 1% penicillin-streptomycin solution (10,000 units/mL penicillin and 10 mg/mL streptomycin; Sigma-Aldrich) between 2-10 x $10^5$ cells/mL at 37 °C in a controlled 5% $CO_2$ atmosphere. THP1 cells were counted by trypan blue (Sigma-Aldrich) exclusion method and the concentration was adjusted to 2 x $10^5$ cells/mL in cell culture medium without antibiotics. Cells were simultaneously differentiated with 20 nM phorbol 12-myristate 13-acetate (PMA; Sigma-Aldrich) and infected at a multiplicity of infection (MOI) of 1:1 with the single bacterial suspension previously prepared. After for 4 hours of incubation with stirring, infected cells were washed five times to remove extracellular bacilli and resuspended in fresh cell culture medium at 4 x $10^5$ cells/mL. Infected cells (50 μl) were seeded at a concentration of 20,000 cells per well in white flat bottom 96-well plates containing serial dilutions of the compounds and nanoparticle samples (50 μl), in a range of concentrations as described in the protocol above. Plates were incubated for 5 days under 5% $CO_2$ atmosphere at 37 °C before the addition of the luciferase substrate D-Luciferin (Perking Elmer) at a final concentration of 1.25 mg/mL as a bacterial growth indicator. Viable M. *tuberculosis* H37Rv-Luc bacteria catalyse the oxidation of D-Luciferin (an ATP-dependent reaction) resulting in the production of light. Luminiscence was measured in a Synergy HT microplate reader (BioTek) 10 min after the addition of D-Luciferin. Internal wells containing drug-free medium with and without infected THP1 cells established maximum and minimal light production, respectively. A 90% reduction in light production was considered growth inhibition. Every compound was assayed in triplicate in at least two independent experiments.

### 2.2. RESULTS

### 2.2.1. Antituberculosis activity of AS-48 containing nanoformulations against extracellular M. tuberculosis

The series of nanoformulations described in Gaglio et al. 2022 (doi: 10.3390/pharmaceutics14122744) were tested *in vitro* for their activity against *M. tuberculosis* extracellular cultures. We first determined the percentage of growth inhibition in the treated samples compared with that of the untreated positive controls (Figure 2).

AS-48, both free and immobilized on BMNPs were able to decrease extracellular *M. tuberculosis* proliferation (Figure 2), indicating the antibacterial activity of these nanoformulations against *M. tuberculosis* outside macrophages. Bacteriocin AS-48 produced a sharp decrease in the proliferation of *M. tuberculosis* at concentrations between 32 and 64 μg/mL, whereas when treating with AS-48_BMNP, such decrease could be observed at higher concentrations, between 90 and 128 μg/mL of immobilized AS-48. This loss of activity of immobilized molecules is common. Although treatment with PLGA[AS-48_BMNPs] resulted in a progressive decline in the ability of *M. tuberculosis* to proliferate growth over untreated cultures could not fall beyond 40%, indicating a significant loss of AS-48 antituberculosis activity in this nanoformulation.

As indicated above, the concentration of bacteriocin AS-48 (free or immobilized) resulting in 90% growth inhibition (10% growth over untreated cultures) was taken as the MICs of each specimen. These results are summarized in Table 2.

**Table 2.** Concentrations of bacteriocin AS-48, PLGA, BMNP and their relevant combinations (PLGA[BMNP], PLGA [AS-48], AS-48_BMNP, and PLGA[AS-48_BMNP]) against cultures of *M. tuberculosis* H37Rv. For the lowest concentration of the combinations resulting in inhibition of bacterial growth, the concentration of each individual component is shown.

| Compound or nanoformulation | MIC ($\mu$g/mL or mg/mL) |
|---|---|
| AS-48 | 45.2 - 64 $\mu$g/mL |
| PLGA | >3.7 mg/mL |
| BMNP | >1.8 mg/mL |
| PLGA[BMNP] | 3.7 mg/mL PLGA; 1.8 mg/mL BMNP |
| PLGA[AS-48] | >3.7 mg/mL PLGA; >256 $\mu$g/mL AS-48 |
| AS-48_BMNP | 0.9 mg/mL BMNP; 128 $\mu$g/mL AS-48 |
| PLGA[AS-48_BMNP] | >3.7 mg/mL PLGA; >1.8 mg/mL BMNP; >256 $\mu$g/mL AS-48 |

From the data shown in Figure 2 and Table 2, it can be concluded that the immobilization of AS-48 to BMNPs only reduced the bacteriocin activity 2-3 x (the MIC increased from 45.2 - 64 $\mu$g/mL for the fee AS-48 to 128 $\mu$g/mL for AS-48_BMNPs). However, the association of bacteriocin AS-48 with PLGA or the embedding of AS-48_BMNPs in this polymer completely abolished its antituberculosis activity *in vitro* against cultures of *M. tuberculosis*. None of the materials used to produce the nanoformulations (BMNPs, PLGA, or their combination) resulted in significant antituberculosis activity even at the maximum concentrations tested (3.7 mg/mL for PLGA, and 1.8 mg/mL for BMNP). The MIC of bacteriocin AS-48 alone was consistent with our previous observations (Aguilar-Perez et al., 2018).

Given that a strong synergism of AS-48 with antituberculosis drug ethambutol was observed (Aguilar-Perez et al., 2018, doi: 10.1128/AAC.00359-18), we tested whether this synergism was maintained when AS-48 was immobilized on BMNPs. In this case we could not detect any change in the MICs of AS-48_BMNP nor PLGA[AS-48_BMNP] regarding the presence or not of ethambutol, hence concluding that no additional drugs (at least, ethambutol) need to administer in this treatment.

### 2.2.2 Antituberculosis activity of AS-48 containing nanoformulations against M. tuberculosis infected macrophages

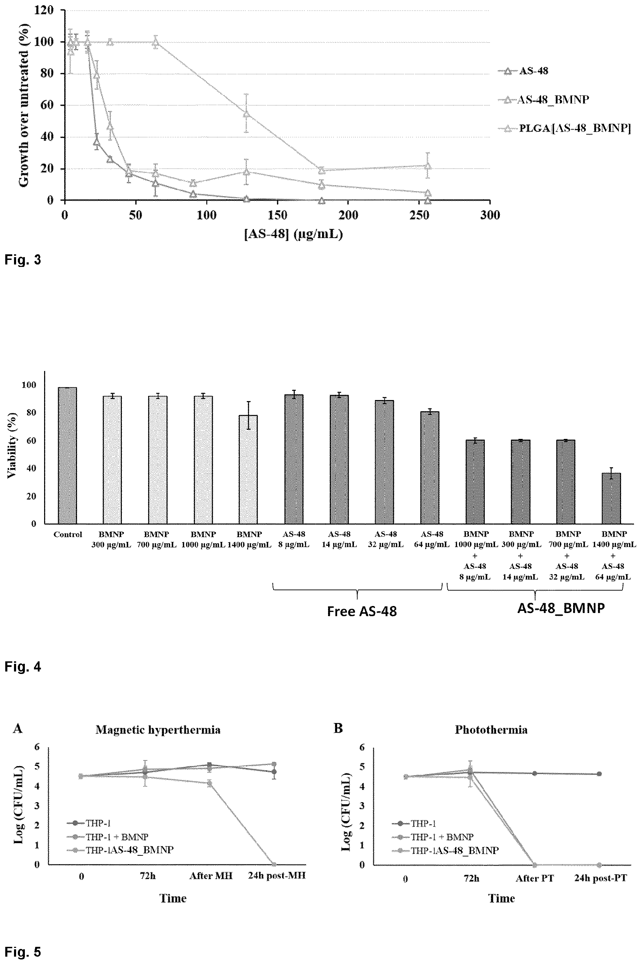

Given our previous observation that BMNPs, PLGA-BMNPs, and PLGA[BMNP+AS-48] were efficiently internalised in THP-1 cells (Gaglio et al., 2022), and that free bacteriocin AS-48 was active against this type of cells when infected by *M. tuberculosis* (Aguilar-Perez et al., 2018, doi: 10.1128/AAC.00359-18), we decided to test next whether these formulations could be active against *M. tuberculosis-infected* THP-1 macrophages. A representative experiment of dose-dependent growth of *M. tuberculosis* H37Rv-Luc cultures in showed in Figure 3.

As occurred with extracellular *M. tuberculosis,* AS-48, both free and immobilized presented antibacterial activity against intracellular *M. tuberculosis.* Free AS-48 caused a drastic reduction on the intracellular growth of *M. tuberculosis* when concentrations below 50 $\mu$g/mL were used. Interestingly, both nanoformulations containing AS-48 (i.e. AS-48_BMNP, and PLGA[AS-48_BMNP]) decreased the growth of intracellular *M. tuberculosis* more than they did in extracellular conditions (Figure 2). Again, AS-48_BMNP nanoformulation showed better antibacterial activity than that of PLGA[AS-48_BMNP], which was not able to completely kill the bacteria under the conditions tested. The concentration at which growth fell below 10% was defined as the MIC in intracellular conditions (Table 3).

**Table 3.** Concentrations of bacteriocin AS-48, PLGA, BMNP and their relevant combinations (PLGA[BMNP], AS-48_BMNP, and PLGA[AS-48_BMNP]) against *M. tuberculosis* H37Rv-Luc strain in infected THP-1 macrophages. For the lowest concentration of the combinations resulting in inhibition of bacterial intracellular growth, the concentration of each individual component in the well is shown.

| Compound or nanoformulation | MIC ($\mu$g/mL or mg/mL) |
|---|---|
| AS-48 | 64 $\mu$g/mL |
| PLGA | >3.7 mg/mL |

(continued)

| Compound or nanoformulation | MIC ($\mu$g/mL or mg/mL) |
| --- | --- |
| BMNP | >1.8 mg/mL |
| PLGA[BMNP] | >3.7 mg/mL PLGA; >1.8 mg/mL BMNP |
| BMNP + AS-48 | 0.45 mg/mL BMNPs; 64 $\mu$g/mL AS-48 |
| PLGA[BMNP+AS-48] | 2.62 mg/mL PLGA; 1.27 mg/mL BMNPs; 181 $\mu$g/mL AS-48 |

From Table 3 and Figure 3, it is clear that AS-48_BMNPs has antibacterial activity against *M. tuberculosis* when this bacterium is infecting macrophages and that the immobilization of AS-48 on BMNP did not affect its antituberculosis activity against intracellular *M. tuberculosis* in a detectable level, since the MIC for both AS-48 and AS-48_BMNP+AS-48 were the same (64 $\mu$g/mL of AS-48). Further encapsulation of BMNP+AS-48 with PLGA increased the MIC of AS-48 against intracellular bacteria to 181 $\mu$g/mL (Table 3) whereas against plain cultures of *M. tuberculosis* this nanoformulation failed to efficiently arrest bacterial growth even at the highest concentration tested (Table 2). No synergic effect with ethambutol was observed in these experiments either.

**CONCLUSIONS**

From our experiments, we can conclude the following:

- Bacteriocin AS-48 immobilized on BMNPs inhibits growth of *M. tuberculosis* in both extracellular and intracellular conditions, making it possible a potential local and directed treatment.
- AS-48_BMNP is efficient for killing intracellular *M. tuberculosis*.
- PLGA[AS-48_BMNP] nanoformulations were inactive against cultures of *extracellular M. tuberculosis,* but were active against *M. tuberculosis* residing in macrophages.

  - Immobilization of bacteriocin AS-48 with BMNPs, with or without PLGA covering, is a promising approach to target intracellular pathogens such as *M. tuberculosis*
  - Combination of nanoformulations containing bacteriocin AS-48 with subinhibitory concentrations of ethambutol does not affect antituberculosis activity of AS-48.

**EXAMPLE 3. EFFICIENCY OF BMNP MEDIATED NANOFORMULATIONS AGAINST *Mycobacterium tuberculosis:* SINERGY WITH HYPERTHERMIA (MAGNETIC HYPERTHERMIA AND PHOTOTHERMIA).**

Another advantage of using AS-48 immobilized on BMNPs is the possible combination of the *tuberculosis* bactericidal action of AS-48 with the damage produced by magnetic hyperthermia (MH) or photothermia (PT) mediated by BMNPs. When the nanoparticles are subjected to alternating magnetic fields (AMF) during MH experiments, they rotate, rising the temperature and damaging cell wall, both by this temperature rise and by mechanical damages, facilitating the entry of AS-48 and its insertion in the bacterial cytoplasmic membrane. In case of PT treatments, BMNPs are exposed to a near infrared laser (NIR) and transform the absorbed energy into heat. In both cases, the thermic increase may enhance the activity of AS-48 against pathogens and/or induce cellular apoptosis mechanisms that end with the elimination of the bacteria replicative niche. Therefore, the combination of AS-48 immobilized on BMNPs with the application of MH or PT emerges as an alternative to conventional tuberculosis treatments, not only because a potential direct damage on bacteria, but also because the alterations in the host cells indirectly may reduce the bacterial survival.

**3.1. MATERIALS AND METHODS**

*3.1.1. Nanoformulations, bacterial and cell cultures*

BMNPs and AS-48_BMNPs were prepared accordingly to Gaglio et al., 2022 (doi: 10.3390/pharmaceutics14122744). Prior to the following assays, the BMNPs or the AS-48_BMNPs nanoassemblies were resuspended in the same culture medium as the cells and vortexed for 30 seconds (3000 rpm). To further disaggregate them, BMNPs were also sonicated for 10 min (35 W). AS-48_BMNP nanoassemblies were vortexed instead for additional 10 seconds (3000 rpm). The nanoassemblies could not be sonicated, in order to prevent the release of AS-48 from the surface of BMNPs.

*Mycobacterium tuberculosis* H37Rv was used to infect human lung macrophages from THP-1 cell line and perform intracellular experiments. The bacterial strain was grown in Middlebrook 7H9 Broth (Difco) supplemented with 10% Albumin-Dextrose-Catalase (ADC, Becton Dickinson), 0.05% Tween 80 (Sigma-Aldrich) and 20 $\mu$g/mL kanamycin. Solid cultures were prepared using Middlebrook 7H10 Agar (Difco) supplemented with 10% ADC and 0.5% glycerol.

THP-1 monocytes (European Collection of Authenticated Cell Cultures 88081201) were cultivated in RPMI Medium 1640 with 1% GlutaMAX (Gibco) and 10% Fetal Bovine Serum (FBS, Gibco). To differentiate the monocytes into macrophages, 10 ng/mL phorbol 12-myristate 13-acetate (PMA, Sigma-Aldrich) were added to the culture for 24 hours. Cells were grown at 37°C, with 5% $CO_2$ and a humidified atmosphere.

### 3.1.2 MIC and cytotoxicity assays

To determine the Minimum Inhibitory Concentration (MIC) of AS-48 and AS-48_BMNP against intracellular *M. tuberculosis,* 20,000 THP-1 monocytes/well were seeded in 96-well plates and differentiated into macrophages. After 24h incubation, the culture medium was removed and 100 $\mu$L of a $10^6$ CFU/mL *M. tuberculosis* H37Rv culture were added to infect the cells (multiplicity of infection: 5). The plates were incubated for 4h (37°C, 5% $CO_2$) and then washed three times with PBS to eliminate extracellular bacteria. AS-48, AS-48_BMNP and BMNPs (control) were diluted in RPMI medium (10% FBS, 1% GlutaMAX), disaggregated as described above, and added to the plate in the following concentration ranges: 2 to 128 $\mu$g/mL for AS-48 or AS-48_BMNP, and 44 to 2800 $\mu$g/mL for BMNPs. After 6 days of incubation (37°C, 5% $CO_2$), macrophages were lysed using 1% Triton and serial decimal dilutions of each well were done in PBS with 0.1% tyloxapol. The dilutions were plated on Middlebrook 7H10 Agar (10% ADC, 0.5% glycerol) and incubated for 14 days (37°C) to determine the bacterial titer in each one.

To test the effect of BMNPs, AS-48 or AS-48_BMNP on the viability of THP-1 macrophages, 20,000 monocytes/well were seeded in 96-well plates and differentiated into macrophages. Then, the medium was withdrawn and 100 $\mu$L of the disaggregated treatments were added in the following concentration ranges: 300 to 1400 $\mu$g/mL of BMNPs, and 8 to 64 $\mu$g/mL of free or immobilized AS-48. After 72h of incubation, the cells were trypsinized, stained with 7-AAD and Annexin V-Allophycocyanin (apoptosis markers), and fixed with 4% paraformaldehyde in DPBS supplemented with $Ca^{2+}$ (Gibco). The samples were analyzed by flow cytometry (Gallios Flow Cytometer, Beckman Coulter), collecting the emission wavelengths at 695 nm (7-AAD) and 660 nm (Annexin V-Allophycocyanin). The data were processed with Kaluza Software (Beckman Coulter).

### 3.1.3. Combined treatments of AS-48_BMNPs with MH or PT

To test the combined therapy of AS-48_BMNPs with MH or PT against infected THP-1 macrophages, 150,000 THP-1 monocytes/well were seeded in 24-well plates and differentiated into macrophages. Cells were infected with *M. tuberculosis* (multiplicity of infection: 5) for 4 hours, and extracellular bacteria were removed by washing the cells with PBS 0.1% tyloxapol. Then, 0.5 mL of the following treatments were added: RPMI medium (control), BMNPs (1 mg/mL), and BMNP+AS-48 (1 mg/mL + 8 $\mu$g/mL). After 72h incubation to guarantee the phagocytosis of the BMNPs or the nanoassemblies, the supernatants were removed and the cells were trypsinized, centrifuged (4000 rpm, 1 min) and resuspended in 100 $\mu$L RPMI. After that, 20 min treatments of MH or PT were applied. MH was conducted by using an alternate current generator at a frequency of 114 kHz and a field strength of 17 kA/m. The equipment consisted of a power supply and a double four-turn copper coil (25 mm diameter), cooled at 15°C by a thermostatic water bath. The samples were placed in the middle of the coil. In case of PT, the samples were placed in 96-well plates and irradiated with a laser (808 nm wavelength) at a power of 1 W/cm$^2$. After the 20 min application of MH or PT, the cells were resuspended in a final volume of 0.5 mL RPMI and incubated for 24h to recover from the treatment. Experiments were conducted in triplicate and aliquots of each sample were taken at the following interval times: 0 (right before adding the treatments), 72h (after the 72h incubation with each treatment), immediately after MH or PT, and 24h post-MH or post-PT (after the 24h recovery period). Half of the aliquots were analyzed by flow cytometry to study the cell viability, and the other half were lysed and plated to determine the number of intracellular CFU at each time interval.

### 3.2. RESULTS

The immobilization of AS-48 on the BMNPs is a simple process that occurs when both elements are mixed at physiological pH. An electrostatic interaction is established between the positive charges of AS-48 and the negative charges present at the surface of the nanoparticles. The adsorption percentage is high (50-80%) and a quantity of 6 to 10 nmol of AS-48 can be immobilized per mg of BMNPs.

The analysis of the antibacterial activity of free and immobilized AS-48 against *M. tuberculosis* (after 6 days of incubation) suggests that the immobilization of the peptide alters its effectiveness. Note that these experiments were performed by using AS-48 form a batch purified differently than in the proof of concept above. Therefore, MIC values are expected to be different than in the previous proof of concept. In the experiments of the present proof of concept, the MIC against *M. tuberculosis* under intracellular conditions is 8 $\mu$g/mL for free AS-48 and 128 $\mu$g/mL for immobilized AS-48. This means that a higher concentration of immobilized AS-48 is necessary to inhibit bacterial growth. However, this increase can be explained by the fact that not all the immobilized peptide is available to attack the plasmatic membrane of the bacteria. BMNPs do not negatively affect the growth of *M. tuberculosis* under any of the tested concentrations.

Cytotoxicity results (Figure 4) indicate that BMNPs are not toxic for THP-1 macrophages at concentrations lower than 1.4 mg/mL (78 $\pm$ 10% viability) and AS-48 is cytocompatible below 64 $\mu$g/mL (81 $\pm$ 2% viability). In contrast, the nanoassemblies AS-48_BMNPs reduce cell viability to 60% or less. This effect on macrophages may be beneficial for the treatment because the destruction of the bacterial replicative niche indirectly favors the elimination of the bacteria. This cytotoxicity in unspecific, and the treatment will kill infected and non-infected macrophages, which highlight the importance of directioning and making the treatment local.

Regarding the combined action of AS-48_BMNPs with MH or PT, results indicate that MH greatly enhances the effect of AS-48 whereas PT is very efficient even in absence of AS-48 (Figure 4). AS-48_BMNPs nanoassemblies (BMNPs 1 mg/mL, AS-48 at 8 $\mu$g/mL) were used in all treatments. Note that the concentration of immobilized AS-48 was 16 times lower than its MIC (128 $\mu$g/mL). This concentration was selected to analyze if MH or PT improved the bactericidal effect allowing to reduce the AS-48 dose.

During the experiment, macrophages were infected (time 0) and incubated for 72 h with AS-48_BMNPs, BMNPs (control to make sure that MT and PT were working right) or culture medium (infection control). After the incubation time, no significant changes in the number of intracellular bacteria were observed. Then, infected macrophages treated as detailed were exposed to alternating magnetic field for MH or to laser irradiation for PT. Immediately after the 20 min application of MH or PT, the bacterial CFU started to decrease. As shown in Figure 5A, the combination of MH with immobilized AS-48 had a bactericidal effect against *M. tuberculosis* that increased with time and that was able to reduce the colony count to 0 CFU/mL. Although BMNPs did not affect the viability of *M. tuberculosis* on their own, when they were exposed to AMF, the induced rotation and temperature increase prompted the local release over time of AS-48, which concentrated at the cell wall and was able to exert its antibacterial activity. Besides, BMNPs rotation exerts a mechanical damage on the bacterial cell wall that facilitates the access of AS-48 to the plasmatic membrane and the formation of pores. Finally, the flow cytometry analyses revealed that after the complete treatment, the macrophages viability was reduced to 40 $\pm$ 20%, which, as previously stated, facilitates the elimination of the infection because of the destruction of the bacterial replicative niche. In case of PT (Figure 5B), the bactericidal effect of BMNPs exposed to laser was able to kill all *M. tuberculosis* population, regardless AS-48.

## CONCLUSIONS

It is interesting to point out that (1) Synergy of the antibacterial treatment mediated by the nanoformulation AS-48_BMNPs with MH and PT was able to reduce the doses of AS-48 while keeping the efficiency of the treatment, and (2) The antibacterial effect was induced immediately after exposure to an AMF or to laser irradiation (in comparison, the time necessary to eradicate the infection at a MIC concentration is 6 days). These results open new opportunities for tuberculosis treatment and could be studied as well with other intracellular pathogens, especially when they are slow-growing bacteria that require long-lasting therapies.

### EXAMPLE 4. EFFICIENCY OF BMNP MEDIATED NANOFORMULATIONS AGAINST BIOFILMS.

#### 4.1. MATERIAL AND METHODS

##### 4.1.1 Nanoformulations

BMNPs and AS-48_BMNPs were prepared accordingly to Jabalera et al., 2021 (doi: 10.1016/j.ijbiomac.2021.08.110). Prior to the following assays, the BMNPs or the AS-48_BMNP nanoassemblies were resuspended in the same culture medium as the biofilms and vortexed for 30 seconds (3000 rpm). To further disaggregate them, BMNPs were also sonicated for 10 min (35 W). AS-48_BMNP nanoassemblies were vortexed instead for additional 10 seconds (3000 rpm). The nanoassemblies could not be sonicated, in order to prevent the release of AS-48 from the surface of BMNPs.

##### 4.1.2 Biofilms

The biofilms were grown on 4 mm diameter sterile glass beads. A bacterial suspension of either *Pseudomonas aeruginosa* PAO-1 or *Enterococcus faecalis* JH2-2 adjusted to $10^8$ UFC/mL was diluted 1:100 in fresh brain heart infusion (BHI) and glass beads on a microtiter plate. It was incubated for 24 h and washed twice with an equal volume of sterile 0.9% NaCl. After that, fresh BHI was added and incubation was prolonged 24 h. Last, the beads were washed again twice with 0.9% NaCl and used in the hyperthermia experiment.

### 4.1.3 Biofilm treatment

Sets of 3 beads with a mature biofilm were incubated: (1) at 37 °C and 45 °C, respectively, serving as temperature control (samples 37 °C and 45 °C, respectively), (2) with 10 mg/mL BMNPs at 37 °C and 45 °C (samples BMNPs 37 °C and BMNPs 45 °C, respectively), (3) with soluble AS-48 at the same concentration as that immobilized on BMNPs (500 $\mu$g/mL AS-48) at 37 °C (sample AS-48 37 °C) and 45 °C (sample AS-48 45 °C) and (4) with AS-48_BMNPs at the same temperatures (samples AS-48_BMNPs 37 °C and AS-48_BMNPs 45 °C). Lastly, the effect of the magnetic hyperthermia caused by the alternating magnetic field (AMF) on the biofilms was tested using BMNPs and AS-48_BMNPs (samples H BMNP and H AS-48_BMNP).

## 4.2. RESULTS

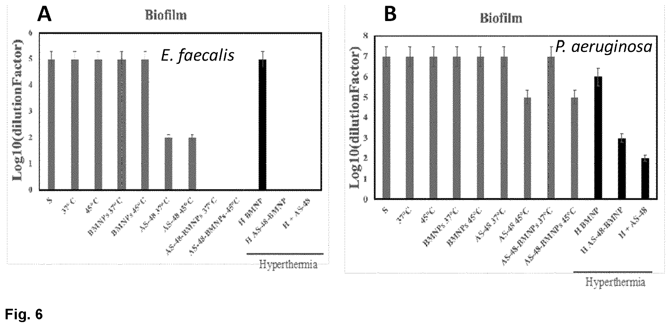

When the nanoassemblies AS-48_BMNPs was applied to biofilms formed by *Enterococcus faecalis* and *Pseudomonas aeruginosa,* chosen as model bacteria, the nanoassembly had a bactericidal effect on both cells released from the biofilm and cells inside the biofilm (Figure 1). *E. faecalis* biofilm viability was not affected by BMNP and/or temperature, but the addition of 500 $\mu$g/mL of AS-48 caused cell death, especially if AS-48 was immobilized on BMNPs. In fact, while the addition of soluble AS-48 decreased *E. faecalis* viability 3-log reduction at either 37 °C or 45 °C, the addition of AS-48_BMNPs caused 5-log reduction of cell viability (Figure 6A). This suggests that AS-48_BMNPs enters the biofilm matrix and releases AS-48, thus having a stronger bactericidal effect. Lastly, AMF-induced hyperthermia showed no effect of BMNPs alone (H BMNP) until AS-48 was added (H+AS-48) which caused a 5-log reduction.

*P. aeruginosa* biofilm viability was less affected by the treatment. As expected, due to its Gram-negative cell wall, AS-48 in solution or attached to BMNPs was poorly active, achieving a 2-log reduction in the biofilm only at increased temperatures (45 °C). This is caused either because of the outer membrane of its cell wall or the protective effect of the biofilm or a combination of both. BMNPs had no effect alone until an alternating magnetic field is applied. In these conditions, a mild effect of naked BMNPs was visible with approximately 1-log reduction in cell viability. 4 log-reduction of cell viability was only achieved when the biofilm was treated with AS-48_BMNPs and exposed to an alternating magnetic field, i.e. following after a combination of an antibacterial therapy and magnetic hyperthermia (Figure 1B). This effect can be put down to the combined mechanical effect of rotation and the added effect of AS-48 since biofilms treated with BMNPs and AMF that were further incubated with 0.5 mg/mL AS-48 at 37 °C for 5 min (H+AS-48 samples) reach 5-log reduction in cell counts compared to those that did not contain AS-48 (H BMNPs).

### CONCLUSIONS

These results confirm thatAS-48_BMNP nanoformulation is able to eradicate biofilms formed by Gram-positive bacteria such as *Enterococcus faecalis* and significantly reduce colony counts in biofilms formed by Gram-negative bacteria such as *Pseudomonas aeruginosa* when combined with hyperthermia. BMNPs mediate a synergic of a directed antibacterial treatment with AS-48 with thermal and mechanical effects induced by the rotation of BMNPs when exposed to an AMF.

### Claims

1. A composition comprising: (i) a substantially pure mineral phase of superparamagnetic magnetite, (ii) the MamC protein, and (iii) optionally, the Mms6 and/or Mms7 protein; wherein, at least components (i) and (ii) form superparamagnetic magnetic nanoparticles comprising up to 20 wt% of MamC, with a mean particle size between 30 and 120 nm, preferably between 30 and 50 nm, more preferably about $39\pm7$ nm,
   wherein the composition is **characterized in that** said superparamagnetic magnetic nanoparticles comprising MamC are disaggregated or dispersed.

2. The composition of claim 1, wherein the superparamagnetic magnetic nanoparticles are bound to one or more therapeutic agents by a non-covalent bond, preferably by electrostatic interaction, to the surface of the superparamagnetic magnetic nanoparticles.

3. The composition of claim 1, wherein the disaggregated or dispersed superparamagnetic magnetic nanoparticles comprising MamC are obtained by subjecting a solution or dispersion comprising the superparamagnetic magnetic nanoparticles at a concentration of less than 100 mg/mL, preferably about 15 mg/mL, to a first cycle of vortex of about 1 second to 30 minutes at 30 to about 9000 rpm, preferably 30 seconds at about 3000 rpm, optionally followed by a sonication step, and preferably followed by a second or subsequent cycle of vortex of about 1 second to 30 minutes at 30 to about 9000 rpm, preferably 30 seconds at about 3000 rpm; wherein the cycles of vortex are carried out at a temperature below 37°C, preferably below 35°C, more preferably below 30°C.

4.  The composition of claim 2, wherein the disaggregated or dispersed superparamagnetic magnetic nanoparticles bound to the one or more therapeutic agents are obtained by subjecting a solution or dispersion comprising the said paramagnetic magnetic nanoparticles at a concentration of less than 100 mg/mL, preferably about 15 mg/mL, to a first cycle of vortex of about 1 second to 30 minutes at 30 to about 9000 rpm, preferably 30 seconds at about 3000 rpm, preferably followed by a second or subsequent cycle of vortex of about 1 second to 30 minutes at 30 to about 9000 rpm, preferably 30 seconds at about 3000 rpm; wherein the cycles of vortex are carried out at a temperature below 37°C, preferably below 35°C, more preferably below 30°C.

5.  The composition of any one of claims 1 or 3, wherein the superparamagnetic magnetic nanoparticles have an isoelectric point of 3-7, preferably 3-5, most preferably of ~4.4.

6.  The composition of any one of claims 1 to 5, wherein the composition is embedded or covered in PLGA or in one or more variants of poly[lactic-co-glycolic acid] (PLGA) or liposomes.

7.  The composition of any one of claims 1 to 6, for use in a method of therapy.

8.  The composition of any one of claims 1 to 6, for use in a method of treatment of microbial infections caused by Gram-positive and/or Gram-negative bacteria or other type of (micro)organisms.

9.  The composition of any one of claims 1 to 5, for use in a method of treatment of intracellular pathogens.

10. The composition of claim 6, for use in a method of treatment of intracellular pathogens.

11. The composition of any one of claims 9 or 10, wherein the composition is as defined in claim 2, and wherein the therapeutic agent is AS-48, and the intracellular agent is *Mycobacterium tuberculosis.*

12. The composition of any one of claims 7 to 11, for use in the treatment method in combination with magnetic hyperthermia (MH) and/or photothermia (PT) mediated by BMNP.

13. The composition of any one of claims 1 to 6, for used in a method of treatment of biofilms formed by Gram-positive or Gram-negative bacteria, preferably in combination with magnetic hyperthermia (MH) and/or photothermia (PT) mediated by BMNP.

14. The composition of claim 13, for used in the treatment method in combination with magnetic hyperthermia (MH) and/or photothermia (PT) mediated by BMNP.

15. A pharmaceutical composition which comprises the composition of any one of claims 1 to 6 and a pharmaceutically acceptable vehicle and/or diluent.

**Fig. 1**

**Fig. 2**

**Fig. 3**

**Fig. 4**

**Fig. 5**

**Fig. 6**

Europäisches
Patentamt

European
Patent Office

Office européen
des brevets

## EUROPEAN SEARCH REPORT

**Application Number**

EP 24 38 2099

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/089505 A1 (UNIV GRANADA [ES]) 7 May 2020 (2020-05-07) * figures 3, 6A, 6B; examples 8-16 * | 1-7,12, 14,15 | INV. A61K9/00 A61K9/51 |
| X,D | JABALERA Y ET AL: "Antibacterial directed chemotherapy using AS-48 peptide immobilized on biomimetic magnetic nanoparticles combined with magnetic hyperthermia", INTERNATIONAL JOURNAL OF BIOLOGICAL MACROMOLECULES, ELSEVIER BV, NL, vol. 189, 19 August 2021 (2021-08-19), pages 206-213, XP086788231, ISSN: 0141-8130, DOI: 10.1016/J.IJBIOMAC.2021.08.110 [retrieved on 2021-08-19] * points 2.3, 2.4, 2.5, 2.6; figures 1, 4 * | 1-15 | ADD. A61K9/10 |

**TECHNICAL FIELDS SEARCHED (IPC)**

A61K

The present search report has been drawn up for all claims

| Place of search | Date of completion of the search | Examiner |
|---|---|---|
| The Hague | 8 July 2024 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 38 2099

This annex lists the patent family members relating to the patent documents cited in the above-mentioned European search report.
The members are as contained in the European Patent Office EDP file on
The European Patent Office is in no way liable for these particulars which are merely given for the purpose of information.

08-07-2024

| Patent document cited in search report | Publication date | Patent family member(s) | Publication date |
|---|---|---|---|
| WO 2020089505 A1 | 07-05-2020 | EP 3875433 A1<br>ES 2758400 A1<br>US 2022143212 A1<br>WO 2020089505 A1 | 08-09-2021<br>05-05-2020<br>12-05-2022<br>07-05-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017153996 A

- EP 3875433 A, Garcia-Rubia

**Non-patent literature cited in the description**

- **EL-BOUBBOU, K**. Magnetic iron oxide nanoparticles as drug carriers: preparation, conjugation and delivery.. *Nanomedicine*, 2018, vol. 13, 929-952
- **GARCIA RUBIA, G** ; **PEIGNEUX, A.** ; **JABALERA, Y** ; **PUERMA, J.** ; **OLTOLINA, F** ; **ELERT, K** ; **COLANGELO, D** ; **GÓMEZ MORALES, J.** ; **PRAT, M** ; **JIMENEZ-LOPEZ, C.** pH-Dependent adsorption release of doxorubicin on MamC-Biomimetic Magnetite Nanoparticles.. *Langmuir*, 2018, vol. 34, 13713-13724
- **UBAGO-RODRIGUEZ, A** ; **CASARES ATIENZA, S** ; **FERNÁNDEZ-VIVAS, A** ; **PEIGNEUX, A** ; **JABALERA, Y** ; **DE LA CUESTA-RIVERO, M** ; **JIMENEZ-LOPEZ, C** ; **AZUAGA FORTES, A.I.** Structure-Function of MamC Loop and Its Effect on the in Vitro Precipitation of Biomimetic Magnetite Nanoparticles. *Crystal Growth and Design*, 2019, vol. 19 (5), 2927-2935
- **VALVERDE-TERCEDOR, C** ; **MONTALBÁN-LÓPEZ, M** ; **PEREZ-GONZALEZ, T.** ; **SANCHEZ-QUESADA, M,S** ; **PROZOROV, T** ; **PINEDA-MOLINA, E** ; **FERNANDEZ-VIVAS, M.A** ; **RODRIGUEZ-NAVARRO, A.B** ; **JIMENEZ-LOPEZ C**. Size control of in vitro synthesized magnetite crystals by the MamC protein of Magnetococcus marinus strain MC-1.. *Appl. Microbiol. Biotechnol.*, 2015, vol. 99, 5109-5121

- **WANG, H.** ; **ZHAO, B.** ; **DONG, W.** ; **ZHONG, Y.** ; **ZHANG, X** ; **GONG, Y** ; **ZHAN, R** ; **XING, M.** ; **ZHANG, J.** ; **LUO, G et al.** A Dual-Targeted Platform Based on Graphene for Synergistic Chemo-Photothermal Therapy against Multidrug-Resistant Gram-Negative Bacteria and Their Biofilms.. *Chemical Engineering Journal*, 2020, vol. 393, 124595
- **WU, B.** ; **FU, J.** ; **ZHOU, Y** ; **SHI, Y** ; **WANG, J.** ; **FENG, X** ; **ZHAO, Y** ; **ZHOU, G.** ; **LU, C.** ; **QUAN, G. et al.** Metal-Organic Framework-Based Chemo-Photothermal Com-binational System for Precise, Rapid, and Efficient Antibacterial Therapeutics.. *Pharmaceutics*, 2019, vol. 11, 463
- Remington's Pharmaceutical Sciences. 1980
- **ANDREU N** ; **ZELMER A** ; **FLETCHER T** ; **ELKINGTON PT** ; **WARD TH** ; **RIPOLL J** ; **PARISH T** ; **BANCROFT GJ** ; **SCHAIBLE U** ; **ROBERTSON BD**. Optimisation of bioluminescent reporters for use with mycobacteria.. *PLoS One.*, 24 May 2010, vol. 5 (5), e10777